(19) Europäisches Patentamt
European Patent Office
Office européen des brevets

(11) **EP 4 651 074 A1**

(12) **EUROPEAN PATENT APPLICATION**
published in accordance with Art. 153(4) EPC

(43) Date of publication:
**19.11.2025 Bulletin 2025/47**

(21) Application number: **23916187.0**

(22) Date of filing: **11.12.2023**

(51) International Patent Classification (IPC):
***G06T 7/00*** (2017.01)   ***A61B 5/055*** (2006.01)

(52) Cooperative Patent Classification (CPC):
**A61B 5/055; G06T 7/00**

(86) International application number:
**PCT/JP2023/044155**

(87) International publication number:
**WO 2024/150572 (18.07.2024 Gazette 2024/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.01.2023 JP 2023002604**

(71) Applicant: **FUJITSU LIMITED**
**Kawasaki-shi, Kanagawa 211-8588 (JP)**

(72) Inventors:
• **KARASUDANI, Ayu**
  **Kawasaki-shi, Kanagawa 211-8588 (JP)**
• **ISHIHARA, Masaki**
  **Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(54) **IMAGE IDENTIFICATION METHOD AND IMAGE IDENTIFICATION PROGRAM**

(57) Input images are identified as images of a specified lesion region with high accuracy.

A computer cuts out, from a tomographic image obtained by imaging the inside of a human body, a plurality of partial images, which include the same position in the tomographic image and have different sizes, calculates a probability of each of the plurality of partial images being a region of a specified lesion, calculates an integrated value by integrating the probabilities calculated from the plurality of partial images using a calculation that increases a contribution of probabilities calculated from partial images corresponding to at least an intermediate size out of the plurality of partial images, and identifies the same position as a region of the specified lesion when the integrated value exceeds a predetermined threshold.

FIG. 7

EP 4 651 074 A1

## Description

Technical Field

The embodiments discussed herein relate to an image identification method and an image identification program.

Background Art

Medical images produced by techniques such as computed tomography (CT) and magnetic resonance imaging (MRI) are widely used to diagnose various diseases. When using medical images for diagnosis, a doctor needs to interpret many images, which places a heavy burden on the doctor. For this reason, there is demand for a computer-based technique that supports diagnosis work by doctors in some way.

The following diagnosis support techniques that use medical images have been proposed. As one example, a proposed medical image processing apparatus includes a first identifier device, which identifies lesion region candidates in a medical image, and a second identifier device, which identifies whether the lesion candidate regions identified by the first identifier device are blood vessel regions, and detects, as lesion regions, lesion candidate regions that have not been identified as blood vessel regions by the second identifier device. Another proposed medical image processing apparatus specifies lesion candidate regions included in a medical image, divides each lesion candidate region into a plurality of divided regions, and extracts feature amounts corresponding to the plurality of divided partial regions.

Citation List

Patent Literature

PTL1: Japanese Laid-open Patent Publication No. 2018-175343
PTL2: Japanese Laid-open Patent Publication No. 2016-7270

Summary of Invention

Technical Problem

However, for an identification process that identifies from a medical image whether a region subjected to imaging is a lesion region, such identification may be difficult depending on the disease. As one example, there are cases where a specified lesion region and a specified part of the body that is normal will appear with similar brightness in a medical image but the three-dimensional shapes of the former lesion region and the latter specified part will differ. In such cases, when the lesion region and the specified part have different shapes in a medical image, it is highly likely that it will be possible to identify both the lesion region and the specified part. However, in some cases, both may appear in a medical image with similar shapes, which may prevent the lesion region and the specified part from being accurately identified.

For the cases described above, the identification accuracy will vary depending on the size with which the lesion region or the specified part appears in the image. For this reason, a method that cuts out a plurality of partial images with different sizes from the same position in a medical image, executes an identification process to identify whether each partial image is a lesion region, and combines the obtained identification results to output a final identification result is conceivable. For this method however, there is the problem of how to combine the identification results obtained for each partial image to obtain the final identification result.

According to one aspect, it is an object of the present embodiments to provide an image identification method and an image identification program capable of accurately identifying whether an input image is an image of a specified lesion region.

Solution to Problem

According to an aspect of the embodiments, there is provided an image identification method including: cutting out, from a tomographic image obtained by imaging of an inside of a human body, a plurality of partial images that include a same position in the tomographic image and have different sizes; calculating a probability of each of the plurality of partial images being a region with a specified lesion; calculating an integrated value by integrating the probabilities calculated from each of the plurality of partial images using a calculation that increases a contribution of probabilities calculated from partial images corresponding to at least an intermediate size out of the plurality of partial images; and identifying the same position as a region with the specified lesion upon determining that the integrated value exceeds a predetermined threshold.

According to another aspect of the present embodiments, there is provided an image identification program that causes a computer to execute processing that is similar to the image identification method described above.

In addition, according to yet another aspect of the present embodiments, there is provided an image identification method including: cutting out, from three-dimensional volume data generated based on a plurality of tomographic images obtained by imaging of an inside of a human body, a plurality of three-dimensional partial regions that include a same position in the volume data and have different sizes; generating, for each of the plurality of three-dimensional partial regions, a plurality of projection images by performing minimum intensity projection or maximum intensity projection of values of voxels in the three-dimensional partial region in a plurality of directions that are perpendicular to each other, and calculating a probability of the same position being a region with a specified lesion based on the generated plurality of projection images; calculating an integrated value by integrating the probabilities calculated from each of the plurality of three-dimensional partial regions using a calculation that increases a contribution of probabilities calculated from three-dimensional partial regions corresponding to at least an intermediate size out of the plurality of three-dimensional partial regions; and identifying the same position as a region with the specified lesion upon determining that the integrated value exceeds a predetermined threshold.

According to another aspect of the present embodiments, there is provided an image identification program that causes a computer to execute processing that is similar to the image identification method described above.

Advantageous Effects of Invention

According to an aspect of the present embodiments, it is possible to identify, with high accuracy, whether an input image is a region of a specified lesion.

Brief Description of Drawings

FIG. 1 depicts an example configuration and example processing of an image identification apparatus according to a first embodiment.
FIG. 2 depicts an example configuration of a diagnosis support system according to a second embodiment.
FIG. 3 depicts an example hardware configuration of an image identification apparatus.
FIG. 4 is a first diagram depicting a comparative example of a lesion identification process.
FIG. 5 is a second diagram depicting a comparative example of a lesion identification process.
FIG. 6 is a third diagram depicting a comparative example of a lesion identification process.
FIG. 7 depicts one example of a lesion identification process according to the second embodiment.
FIG. 8 depicts one example of a training patch generation process.
FIG. 9 is a first diagram depicting one example of a score integration process.
FIG. 10 is a second diagram depicting one example of the score integration process.
FIG. 11 is a third diagram depicting one example of the score integration process.
FIG. 12 is a fourth diagram depicting one example of the score integration process.
FIG. 13 depicts an example configuration of processing functions included in the training processing apparatus and the image identification apparatus.
FIG. 14 is an example flowchart depicting a procedure of a training process by the training processing apparatus.
FIG. 15 is an example flowchart depicting the procedure of a training patch generation process.
FIG. 16 is an example flowchart depicting the procedure of an image identification process performed by the image identification apparatus.
FIG. 17 is an example flowchart depicting the procedure of a score calculation process.
FIG. 18 is an example flowchart depicting the procedure of an identification result output process.
FIG. 19 is a first diagram useful in explaining a combined patch generation process.
FIG. 20 is a second diagram useful in explaining a combined patch generation process.
FIG. 21 is an example flowchart depicting the procedure of a training patch generation process.
FIG. 22 is an example flowchart depicting the procedure of a score calculation process.
FIG. 23 is an example flowchart depicting the procedure of an identification result output process.

Description of Embodiments

Preferred embodiments of the present disclosure will be described below with reference to the accompanying drawings.

[First Embodiment]

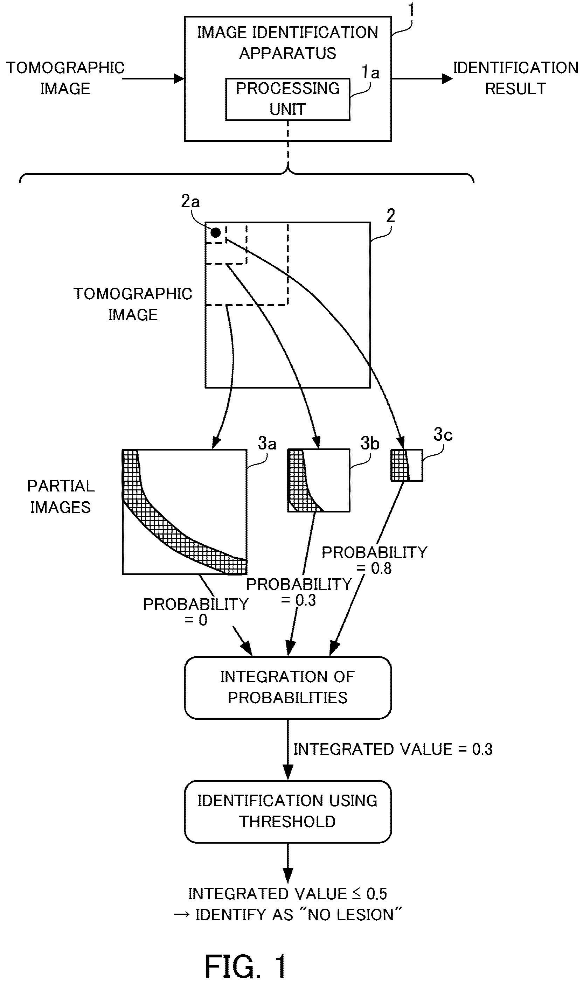

FIG. 1 depicts an example configuration and example processing of an image identification apparatus according to a first embodiment. The image identification apparatus 1 depicted in FIG. 1 is an information processing apparatus that receives an input of a tomographic image obtained by imaging of the inside of a human body and identifies whether each partial region obtained by dividing the tomographic image is a region including a specified lesion. As one example, when a tomographic image of a region including the liver is captured, the image identification apparatus 1 identifies whether each partial region is a tumor region or a normal region. As examples, the tomographic image referred to here is a medical image such as a CT image or an MR image.

Here, there are cases where a lesion region and a specified part of the body that is normal appear with similar brightness in a partial image but the three-dimensional shapes of the former lesion region and the latter specified part differ. In such cases, when the lesion region and the specified part appear in partial images with different shapes, it is highly likely that it will be possible to identify the lesion region and the specified part. However, in some cases, a lesion region and a specified part may appear with similar shapes in a partial image. When this happens, there is the risk of a lesion region and a specified part not being accurately identified.

On the other hand, when the sizes of partial images cut out from the tomographic images are different, the sizes of a lesion region and/or a specified part appearing in the partial images may also differ. As described above, since the lesion region and the specified part have different three-dimensional shapes, when the size of the partial images cut out from the tomographic image is changed, there are cases where the features of the shapes are likely to appear and not likely to appear in partial images. For this reason, when the sizes of the partial images are different and the sizes of a lesion region and/or a specified part captured in the partial images also differ, discrepancies may occur in the identification accuracy for a lesion region.

For this reason, the image identification apparatus 1 cuts out a plurality of partial images with different sizes from a tomographic image at the same position on the tomographic image. The image identification apparatus 1 calculates a probability of each of the plurality of cut-out partial images being a lesion region, and integrates the calculated probabilities to finally identify whether the same position is a lesion region.

The image identification apparatus 1 includes a processing unit 1a. As one example, the processing unit 1a is a processor. The processing unit 1a executes the processing described below.

The processing unit 1a cuts out, from an inputted tomographic image 2, a plurality of partial images that include the same position on the tomographic image 2 and are of different sizes. In the example in FIG. 1, partial images 3a to 3c of three sizes that include the position 2a on the tomographic image 2 are cut out. In this example, the size of the partial image 3a is the largest, the size of the partial image 3b is smaller than the partial image 3a, and the size of the partial image 3c is smaller than the partial image 3b. It is also assumed that the partial images 3a to 3c include the specified part that is normal, mentioned above. Note that as one example, the plurality of partial images may be cut out as images of different sizes centered on the same position.

The processing unit 1a separately calculates the probability of each of the cut-out partial images 3a to 3c being a lesion region. As one example, this calculation is executed using a trained model that identifies whether the input image is a lesion region. In the example in FIG. 1, it is assumed that the probability takes a value from 0 to 1. It is assumed here that the probability is calculated as "0" from the partial image 3a, the probability is calculated as "0.3" from the partial image 3b, and the probability is calculated as "0.8" from the partial image 3c.

The processing unit 1a integrates the probability values calculated from the partial images 3a to 3c to calculate an integrated value. When the calculated integrated value exceeds a predetermined threshold, the processing unit 1a identifies the position 2a as a lesion region. In the present embodiment, it is assumed that the threshold is "0.5", for example.

Here, as one example of an integration process for probabilities, a method of calculating a highest value out of the probabilities calculated from the partial images 3a to 3c as the integrated value is conceivable. However, with this method, there is the risk of a normal region (as one example, a region including the normal specified part mentioned earlier) being erroneously identified as a lesion region. As one example, in FIG. 1, since the highest value of the probability is "0.8" and the calculated integrated value exceeds the threshold "0.5", the position 2a is erroneously identified as a lesion region.

On the other hand, the processing unit 1a of the present embodiment calculates the integrated value using a calculation that increases the contribution of probabilities calculated from partial images, out of the partial images 3a to 3c, corresponding to at least an intermediate size. In the example in FIG. 1, the partial image corresponding to an intermediate size is the partial image 3b, and calculation is performed so that the contribution of the probability "0.3" calculated from the partial image 3b is high. Accordingly, the likelihood of the calculated integrated value not exceeding the threshold "0.5" increases, which increases the likelihood of the position 2a being identified as a normal region.

On the other hand, assume here for example that a lesion region appears in the region of the partial image 3c with the same area as the specified part in FIG. 1. In this case, the likelihood of a probability exceeding the threshold "0.5" being calculated for the partial images 3b and 3c increases. On the other hand, for the partial image 3a, since the lesion region is too small in relative terms, the probability is highly likely to be a relatively low value. For this reason, the integrated value is calculated by way of a calculation where the contribution of the probability calculated from the partial region corresponding to the intermediate size is increased, which increases the likelihood of the region being correctly identified as a lesion region.

As described above, with the image identification apparatus 1 according to the present embodiment, it is possible to accurately identify whether an input image is an image of a specified lesion region.


[Second Embodiment]

Next, a system capable of detecting a tumor in the liver as an example of a lesion will be described.

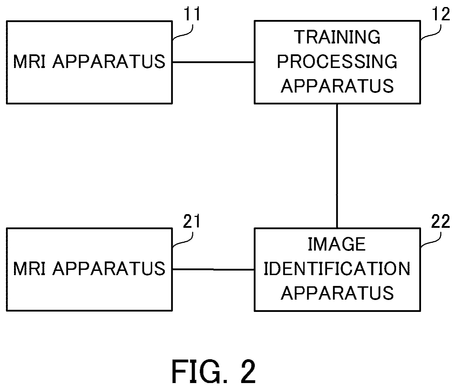

FIG. 2 depicts an example configuration of a diagnosis support system according to a second embodiment. The diagnosis support system depicted in FIG. 2 is a system for supporting image diagnosis work using MRI images, and includes MRI apparatuses 11 and 21, a training processing apparatus 12, and an image identification apparatus 22. Note that the image identification apparatus 22 is one example of the image identification apparatus 1 depicted in FIG. 1.

The MRI apparatuses 11 and 21 capture MR images of a human body. In the present embodiment, the MRI apparatuses 11 and 21 capture a predetermined number of tomographic images on an axial plane in the abdominal region including the liver while changing the position (or "slice position") in the height direction (a direction perpendicular to the axial plane) of the human body at predetermined intervals. In the present embodiment, it is assumed that the MRI apparatuses 11 and 21 perform imaging using a liver contrast agent containing gadoxetate sodium (Gd-EOB-DTPA, EOB: Ethoxybenzyl, DTPA: Diethylenetriamine Penta-acetic Acid) as an active ingredient, and use hepatocyte contrast phase. This contrast agent is hereinafter referred to as an "EOB contrast agent".

The image identification apparatus 22 detects a lesion region from each tomographic image captured by the MRI apparatus 21. In the present embodiment, it is assumed that a tumor in the liver is detected as a lesion region. The image identification apparatus 22 detects lesion regions using a lesion identification model generated by machine learning. As one example, the image identification apparatus 22 has a display apparatus display information indicating an identification result for lesion regions. By doing so, the image identification apparatus 22 supports image diagnosis work by a user (as one example, a radiologist).

The training processing apparatus 12 uses machine learning to generate the lesion identification model used by the image identification apparatus 22. As the model generation process, the training processing apparatus 12 generates training data from each tomographic image captured by the MRI apparatus 11 and executes machine learning using the generated training data. Data (model parameters) indicating the lesion identification model generated by the training processing apparatus 12 is read into the image identification apparatus 22 via a network or a portable recording medium, for example.

Note that captured images may be inputted from the same MRI apparatus into the training processing apparatus 12 and the image identification apparatus 22. The training processing apparatus 12 may acquire captured images via a recording medium or the like instead of directly acquiring the captured images from an MRI apparatus. In addition, the training processing apparatus 12 and the image identification apparatus 22 may be the same information processing apparatus.

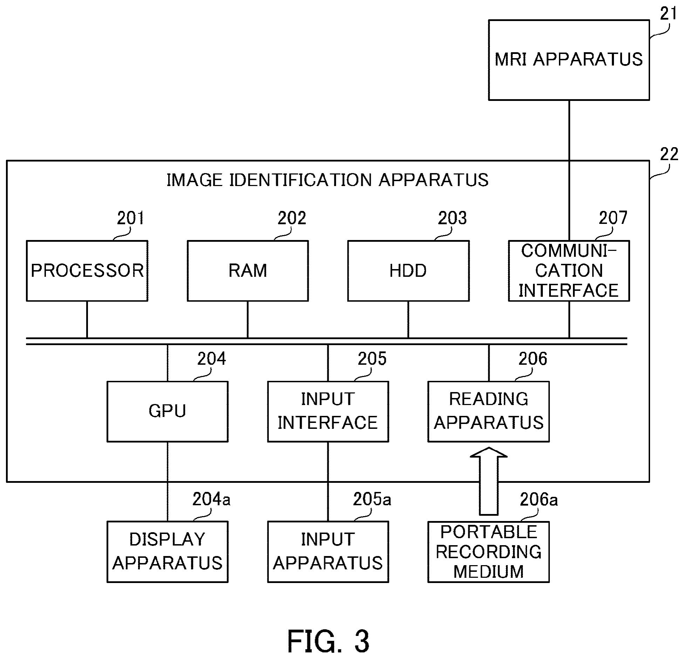

FIG. 3 depicts an example hardware configuration of an image identification apparatus. The image identification apparatus 22 is realized as a computer for example, as depicted in FIG. 3. As depicted in FIG. 3, the image identification apparatus 22 includes a processor 201, a random access memory (RAM) 202, a hard disk drive (HDD) 203, a graphics processing unit (GPU) 204, an input interface 205, a reading apparatus 206, and a communication interface 207.

The processor 201 is in overall control of the entire image identification apparatus 22. As examples, the processor 201 is a central processing unit (CPU), a micro processing unit (MPU), a digital signal processor (DSP), an application specific integrated circuit (ASIC), or a programmable logic device (PLD). The processor 201 may be a combination of two or more elements out of a CPU, an MPU, a DSP, an ASIC, and a PLD. The processor 201 is one example of the processing unit 1a depicted in FIG. 1.

The RAM 202 is used as a main storage device of the image identification apparatus 22. The RAM 202 temporarily stores at least part of an operating system (OS) program and application programs to be executed by the processor 201. The RAM 202 also stores various data that is needed by processing by the processor 201.

The HDD 203 is used as an auxiliary storage device of the image identification apparatus 22. The HDD 203 stores the OS program, application programs, and various data. Note that another type of nonvolatile storage device, such as a solid state drive (SSD), may be used as the auxiliary storage device.

A display apparatus 204a is connected to the GPU 204. The GPU 204 causes the display apparatus 204a to display images in accordance with instructions from the processor 201. Examples of the display apparatus 204a include a liquid crystal display and an organic electroluminescence (EL) display.

An input apparatus 205a is connected to the input interface 205. The input interface 205 transmits a signal outputted from the input apparatus 205a to the processor 201. Examples of the input apparatus 205a include a keyboard and a pointing device. Examples of the pointing device include a mouse, a touch panel, a tablet, a touch pad, and a track ball.

A portable recording medium 206a is detachably attached to the reading apparatus 206. The reading apparatus 206 reads data recorded on the portable recording medium 206a and transmits the data to the processor 201. Examples of the portable recording medium 206a include an optical disc and a semiconductor memory.

The communication interface 207 transmits and receives data to and from another apparatus, such as the MRI apparatus 21, via a network.

The processing functions of the image identification apparatus 22 may be realized using a hardware configuration like that described above. The training processing apparatus 12 may also be realized as a computer with a hardware configuration like that depicted in FIG. 3.

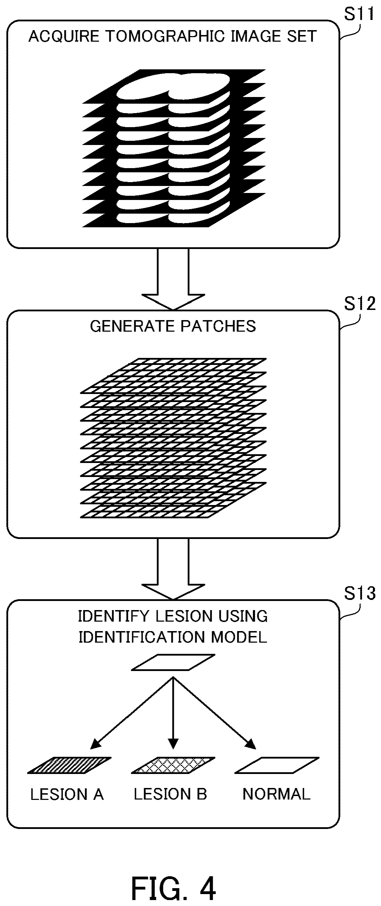

Next, a comparative example of a lesion identification process will be described with reference to FIGS. 4 to 6.

FIG. 4 is a first diagram depicting a comparative example of a lesion identification process. As a method for identifying lesions, there is a method that uses a lesion identification model generated by machine learning. As one example, as depicted in FIG. 4, there is a method of cutting out "patches", which are image regions of a certain size, from each tomographic image and executing lesion identification processing in units of the patches.

In the example in FIG. 4, a tomographic image set is acquired from an MRI apparatus (step S11), and patches are generated from each tomographic image included in the tomographic image set (step S12). The patches are generated by dividing the tomographic image by a certain size, such as 16 pixels by 16 pixels.

The patches generated in this way are inputted into a lesion identification model that has been generated in advance by machine learning. By doing so, any lesions in patches are identified (step S13). In the example in FIG. 4, one of "lesion A", "lesion B", and "normal" (that is, a state with no lesions) is identified by the lesion identification model. In reality, a score indicating the probability of belonging to that particular class is calculated for each of "lesion A", "lesion B", and "normal". When the score corresponding to a certain class exceeds a predetermined threshold, it is determined that the data belongs to that class.

Training data for generating a trained model is also generated as patches cut out from the tomographic image in the same procedure as described above. That is, a label indicating any one of "lesion A", "lesion B", and "normal" is added to training patches and machine learning is performed using such training patches to which labels have been added to generate the lesion identification model.

In the image identification apparatus 22 according to the present embodiment, a lesion identification model that inputs an MR image, which uses an EOB contrast agent, as a tomographic image and identifies whether the image is a tumor (one example of a lesion) or a normal image (that is, not a tumor) is used. Here, in an MR image that uses an EOB contrast agent, a tumor in an organ (here, the liver which appears in the hepatocyte phase) will appear darker compared to the organ, but blood vessels in the organ will similarly appear darker compared to the organ. For this reason, when a tumor is identified using the method in FIG. 4, there is the risk of blood vessel regions (that is, regions that are normal) being erroneously identified as regions of a tumor.

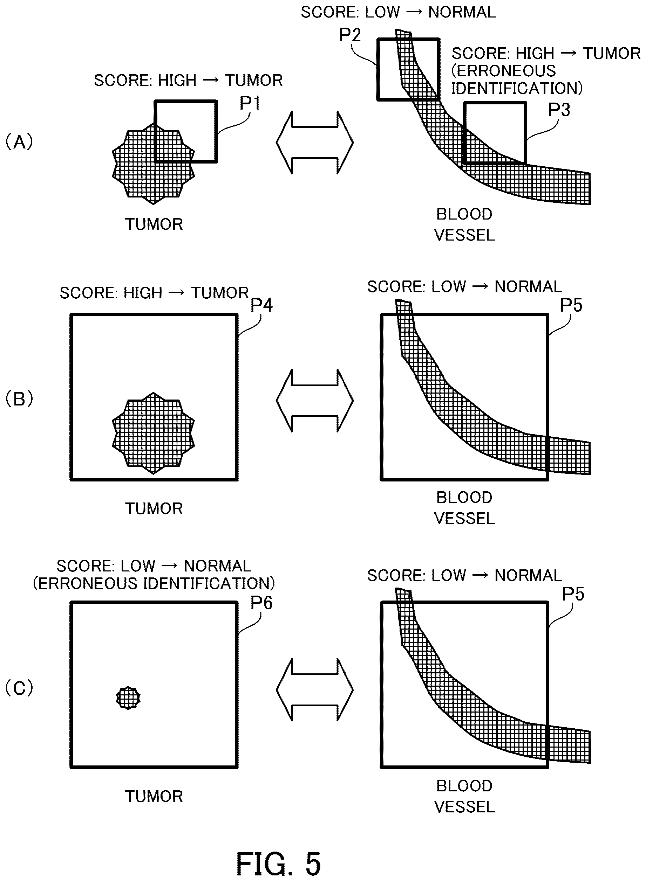

FIG. 5 is a second diagram depicting a comparative example of a lesion identification process. FIG. 5(A) depicts example identification for a case where small patches are used, FIG. 5(B) depicts example identification for a case where large patches are used, and FIG. 5(C) depicts another example identification for a case where large patches are used.

In FIG. 5(A), part of a tumor appears with a certain size or larger in patch P1. When such patch P1 is inputted into the lesion identification model, a relatively high value is calculated as the score indicating the probability of a tumor, so that patch P1 is correctly identified as a tumor.

In patch P2, a blood vessel appears with a tubular shape. When such patch P2 is inputted into the lesion identification model, a relatively low score is calculated, so that the patch P2 is correctly identified as normal. That is, in cases such as patch P2, erroneous identification is unlikely to occur due to the difference in shape from a tumor.

However, patch P3 is disposed so as to graze part of a blood vessel. For this reason, the characteristic tubular shape of a blood vessel does not appear in patch P3. In addition, in patch P3, the region of the blood vessel is depicted with a similar area to the tumor region in patch P1. When such a patch P3 is inputted into the lesion identification model, there is the risk of a similar score to patch P1 being calculated. When this happens, the patch P3 is erroneously identified as a tumor.

FIG. 5(B) depicts example identification when patches P4 and P5, which are larger than patches P1 to P3, are used. A tumor appears in patch P4, and a relatively high score is calculated. For this reason, patch P4 is correctly determined as a tumor. In patch P5, the blood vessel appears with a tubular shape, and a relatively low score is calculated. For this reason, patch P5 is correctly determined to be normal.

When a small patch is used as depicted in FIG. 5(A), there is the risk of erroneous identification occurring depending on the position of the patch relative to a blood vessel. On the other hand, when a large patch is used as depicted in FIG. 5(B), the characteristic shape of a blood vessel is likely to appear in the patch, and erroneous identification is unlikely to occur.

However, it is not the case that erroneous identification is less likely to occur as the patch becomes larger. As one example, in FIG. 5(C), a small tumor is depicted in patch P6 which has the same size as patches P4 and P5. In this case, since the size of the tumor is too small, a relatively low score may be calculated. In this case, a tumor is erroneously identified as normal.

As described above, when the size of the patch changes, the way in which erroneous identification occurs also changes. A patch of a certain size may be unlikely to be erroneously identified as one type of identification category, but may conversely be likely to be erroneously identified as another type of identification category. That is, the appropriate patch size may differ for each type of identification category, such as a tumor or a blood vessel, and it is not possible to know an appropriate patch size for lesion identification in advance.

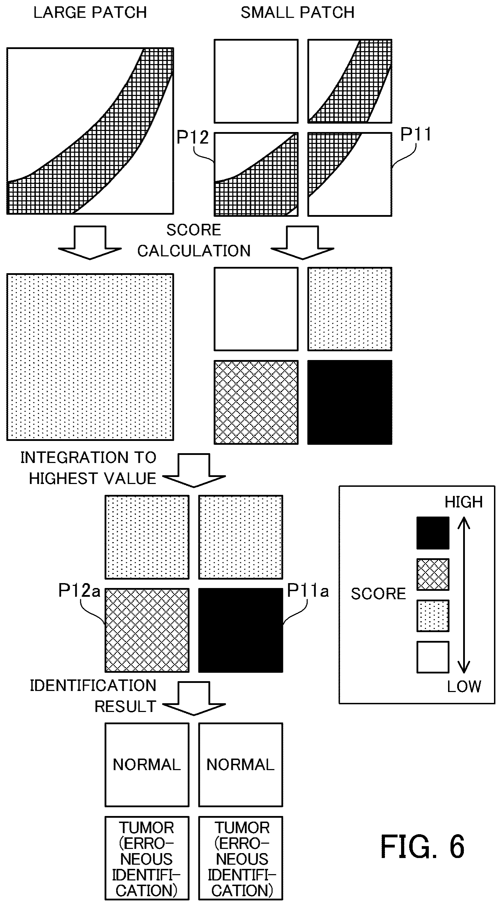

In response to this problem, a method of performing lesion identification using patches of a plurality of sizes (a plurality of scales), integrating identification results of such patches, and outputting a final result would be conceivable. This method may also be referred to as a "multiscale method". Example identification using this multiscale method is depicted in FIG. 6.

FIG. 6 is a third diagram depicting a comparative example of a lesion identification process. FIG. 6 depicts example identification in which a patch (referred to here as a "large patch") of a certain size and a patch (referred to here as a "small patch") whose vertical and horizontal sizes are half the size of the large patch are used. In addition, the score used when each patch is inputted into the lesion identification model is indicated by a value on four levels.

Note that a lesion identification process using large patches and a lesion identification process using small patches may be executed using the same lesion identification model. As one example, assume that a lesion identification model that has been trained using patches of the same size as the large patch is used. In this case, large patches may be directly inputted into the lesion identification model. On the other hand, small patches may be enlarged to the same size as the large patches and such enlarged small patches may be inputted into the lesion identification model.

As depicted in FIG. 6, scores are calculated using the lesion identification model for one large patch and for four small patches. The scores are then integrated between each small patch and a region of the large patch corresponding to that small patch. Note that as an example of actual processing, the large patch is divided into small patches and the score of the large patch is associated with each of these divided patches (score interpolation). The scores are then integrated between the divided patches and the small patches corresponding to the positions of the divided patches.

As one example of the score integration method, a method of calculating the highest value of the score of each patch to be integrated is conceivable. In this method, when the score is high in at least one of the patches that are of different scales, the patch is identified as a tumor. As one example, a tumor that appears small in a large patch may appear with an appropriate size in a small patch and a high score may be calculated for the small patch. This means by integrating the highest values, the likelihood of a small tumor being detected is increased.

However, when a blood vessel appears as depicted in FIG. 6, the blood vessel may be erroneously identified as a tumor. As one example, part of the blood vessel appears in the small patches P11 and P12, but the characteristic tubular shape of a blood vessel does not clearly appear. For this reason, relatively high scores are calculated for the small patches P11 and P12. In this case, when the scores are integrated using the highest value between the large patch and the small patch, relatively high scores are calculated for the integrated patches P11a and P12a corresponding to the small patches P11 and P12, respectively. As a result, the patches P11a and P12a are erroneously identified as tumors. The above problem also occurs when patches of three or more sizes are used.

For this reason, the image identification apparatus 22 according to the present embodiment executes the lesion identification process using patches of three or more sizes, and then executes a different score integration process to that described above.

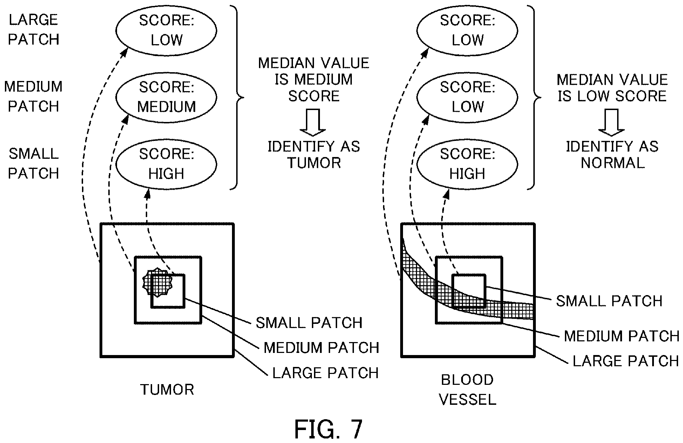

FIG. 7 depicts one example of a lesion identification process according to the second embodiment. FIG. 7 depicts a case where tumors and blood vessels are identified using patches of three sizes, that is, small patches, medium patches, and large patches. Note that as the patch sizes, the following pattern may be used. As one example, the size of the small patch is a region of 6 mm by 6 mm in real space, the size of the medium patch is a region of 12 mm by 12 mm in real space, and the size of the large patch is a region of 24 mm by 24 mm in real space.

There is a characteristic whereby the tendency for the score to differ depending on the patch size differs between tumors and blood vessels. In more detail, a tumor will produce a high score with a patch of an appropriate size and will produce a moderate score with patches of sizes that are relatively close, that is, slightly larger than or slightly smaller than the appropriate size. On the other hand, for blood vessels, in many cases, a patch with a small size in which the blood vessel appears large in the image will produce a high score (that is, will be erroneously identified) and a patch with a larger size in which the blood vessel appears tubular will have a low score.

FIG. 7 depicts a case where erroneous identification is likely to occur when the lesion identification process is executed with small patches. In this example, the tumor appears with an appropriate size in the small patch, so that a high score is calculated from the small patch. In addition, since the size of the tumor is smaller than the appropriate size in the middle patch, a moderate score is calculated from the middle patch. Since the size of the tumor is too small in the large patch, a low score is calculated from the large patch.

On the other hand, it is assumed that the blood vessel depicted in FIG. 7 has a substantially equal width to the diameter of a tumor. In this case, since the small patch includes part of the blood vessel in a state where the characteristic shape of the blood vessel does not appear, a high score is calculated from the small patch. That is, erroneous identification occurs. In addition, since the medium patch and the large patch include part of the blood vessel in a state where the characteristic shape of the blood vessel appears, a low score is calculated from the medium patch and the large patch.

In view of the above characteristics, the image identification apparatus 22 according to the present embodiment integrates scores between patches of different sizes using a calculation method where a tumor is determined when scores that exceed a threshold have been calculated for a plurality of patches of similar sizes. In the example of FIG. 7, since relatively high scores are calculated for the tumor from the small patch and the medium patch, a tumor is correctly identified. On the other hand, since a high score is calculated for the blood vessel from only the small patch and low scores are calculated from the medium patch and the large patch, the region is correctly identified as normal. According to this score integration process, for patches including blood vessels, since calculation is performed so as to suppress the score for the size where erroneous identification occurs, it is possible to reduce the likelihood of a blood vessel being erroneously identified as a tumor.

The following method may be adopted as a specific calculation method of integrating scores. Here, a process F for calculating the integrated score S is defined as $S = F(s_{i-k}, s_i, s_{i+k})$. In this process F, $s_i$ indicates the score of a patch of an $i^{th}$ size currently in focus. The variable k is an arbitrary natural number and is used to designate a patch size that is k patches away from the $i^{th}$ size when the patch sizes are arranged in ascending order. That is, $s_{i-k}$ indicates the score of a patch that is k smaller sizes than the $i^{th}$ size and $s_{i+k}$ indicates the score of a patch that is k sizes larger than the $i^{th}$ size. When there are three patch sizes, k = 1. When the number of patch sizes is four or more, k is an arbitrary natural number that is smaller than 1/2 (the upper limit value) of the number of patch sizes (that is, the number of scales). In the latter case, k may be set by the user, for example, but is preferably a value that is not too close to the upper limit value mentioned above.

As a calculation formula indicating the process F, as examples, any one of the following Equations (1) to (4) may be used.

$$F(s_{i-k}, s_i, s_{i+k}) = \text{median}(s_{i-k}, s_i, s_{i+k}) \quad \cdots (1)$$

$$F(s_{i-k}, s_i, s_{i+k}) = \max(\min(s_{i-k}, s_i), \min(s_i, s_{i+k})) \quad \cdots (2)$$

$$F(s_{i-k}, s_i, s_{i+k}) = s_{i-k}/4 + s_i/2 + s_{i+k}/4 \quad \cdots (3)$$

$$F(s_{i-k}, s_i, s_{i+k}) = s_i/2 + \max(s_{i-k}, s_{i+k})/2 \quad \cdots (4)$$

Here, "median $(x_1, x_2, x_3, \cdots)$" indicates a median value (the value at the middle position) when $x_1$, $x_2$, $x_3$, and $\cdots$ are arranged in ascending order or descending order. "max (x,y)" indicates the larger value out of x and y, and "min(x,y)" indicates the smaller value out of x and y.

In any of Equations (1) to (4) given above, calculation is effectively performed so that the contribution of the score of an intermediate patch size is high. For example, in Equation (3), a large weighting is given to the score of an intermediate patch size. For the case of blood vessels, since it is unlikely that the score will be high only for the intermediate patch size, the likelihood of a blood vessel being erroneously identified as a tumor is reduced by Equation (3).

In addition, with Equation (1), the score of the intermediate patch size is likely to be outputted as an intermediate value. This is because, as described above, in the case of blood vessels, it is unlikely that the score will be high only for the middle patch size. As one example, when Equation (1) is applied to the case depicted in FIG. 7, the median value of the scores of the tumor is an intermediate value, so that the region is identified as a tumor. On the other hand, for the blood vessel, the median value in the scores is a low value and the region is identified as normal.

With Equations (2) and (4), when the scores are high for both an intermediate patch size and any patch size close to this patch size, the integrated score is highly likely to increase. The reason for this is again as described above, for blood vessels, there is a low likelihood that the score will be high only for the intermediate patch size.

As described above, with the image identification apparatus 22 according to the present embodiment, it is possible to reduce the likelihood of erroneously identifying a blood vessel as a tumor while improving the detection accuracy of tumors, and to improve the lesion detection accuracy.

In addition, as described above, there is a characteristic in that the tendency of the score to differ depending on the patch size differs between tumors and blood vessels. To make this characteristic appear more prominently in the lesion identification process, it is desirable to create training data for machine learning of the lesion identification model as follows.

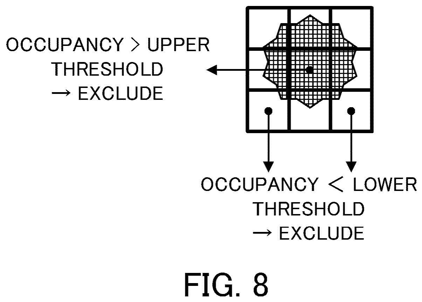

FIG. 8 depicts one example of a training patch generation process. When generating a training patch to which a tumor label is assigned (or "tumor patch"), the training processing apparatus 12 first divides a tomographic image into patches, and specifies a patch in which a tumor appears out of the divided patches as a tumor patch candidate. Next, the training processing apparatus 12 calculates, for each tumor patch candidate, a ratio (or "occupancy") of an area of a region in which the tumor appears out of the entire region of the patch. The training processing apparatus 12 then determines, as tumor patches, only patches whose occupancy is equal to or higher than a predetermined lower limit threshold $TH_{min}$ and equal to or lower than a predetermined upper limit threshold $TH_{max}$ out of the tumor patch candidates, and excludes other patches.

The range from the lower limit threshold $TH_{min}$ to the upper limit threshold $TH_{max}$ indicates a range in which the size of the tumor appearing in a patch is determined to be appropriate. As one example, a tumor has a substantially circular shape on a tomographic image. For this reason, the training processing apparatus 12 calculates the lower limit threshold $TH_{min}$ using Equation (5a) below on the basis of the area of a circle that fits in a square with sides that are half the width of the patch, and calculates the upper limit threshold $TH_{max}$ using Equation (5b) below on the basis of the area of a circle that fits in the patch.

$$TH_{min} = \pi(1/4)^2 \cdots (5a)$$

$$TH_{max} = \pi(1/2)^2 \cdots (5b)$$

The lower limit threshold $TH_{min}$ and the upper limit threshold $TH_{max}$ may also be calculated based on three-dimensional image data based on a plurality of captured tomographic images. As one example, the training processing apparatus 12 cuts out, from three-dimensional volume data generated based on a plurality of tomographic images, a cubic block that has a patch on one surface or on an inside (as one example, at the center). Since a tumor is substantially spherical, the training processing apparatus 12 calculates the lower limit threshold $TH_{min}$ using Equation (6a) below based on the volume of a sphere that fits in a cube with sides that are half the side of the block, and calculates the upper limit threshold $TH_{max}$ using Equation (6b) below based on the volume of a sphere that fits in the block.

$$TH_{min} = (4/3)\pi(1/4)^3 \cdots (6a)$$

$$TH_{max} = (4/3)\pi(1/2)^3 \cdots (6b)$$

As one example, in actual processing when Equations (6a) and (6b) are used, the value of the Equation (6a) is around 0.065 and the value of Equation (6b) is around 0.524, so that $TH_{min}$ = 6% and $TH_{max}$ = 52% may be used.

The training processing apparatus 12 performs machine learning using the tumor patches determined as described above as training data, and generates a lesion identification model. By doing so, it is possible to generate a lesion identification model in which a high score is calculated from a patch in which a tumor is captured with an appropriate area, but a lower score is calculated from a patch in which the area of the tumor is too small or too large. By executing the score integration process depicted in FIG. 7 using this lesion identification model, it is possible to reduce the likelihood of erroneously identifying a blood vessel as a tumor while increasing the detection accuracy of tumors, and to improve the lesion detection accuracy.

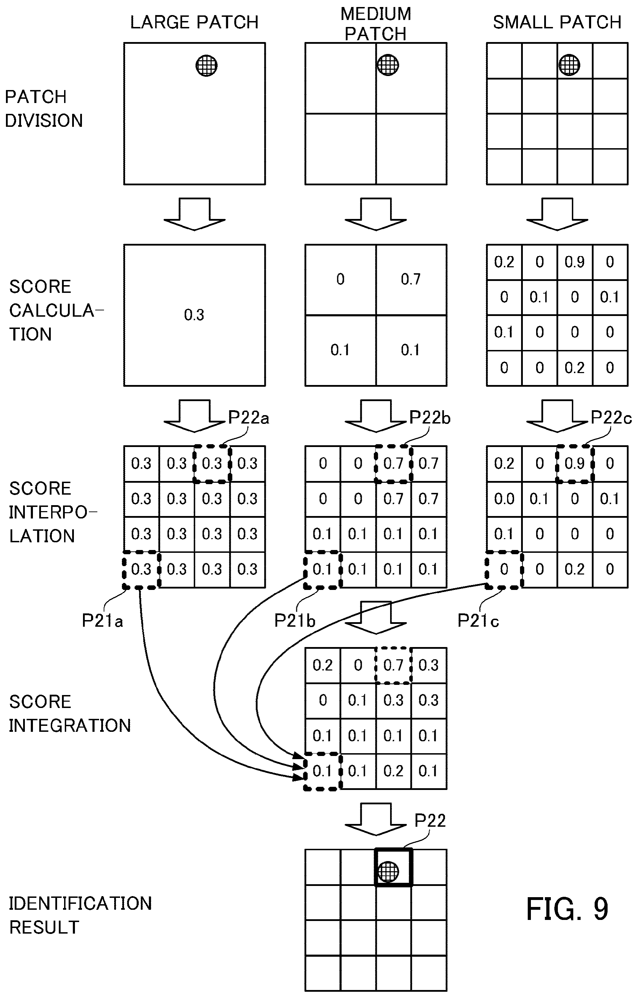

Next, examples of the score integration process will be described with reference to FIGS. 9 to 12. In FIGS. 9 to 12, as one example, it is assumed that the scores are integrated using Equation (1) described earlier.

FIG. 9 is a first diagram depicting one example of a score integration process. FIG. 9 depicts a score integration process for an image region in which a tumor is captured. It is assumed that the score takes a value from 0 to 1 and the threshold for determining a tumor is 0.5.

The image identification apparatus 22 divides a tomographic image into large patches, medium patches, and small patches. Next, the image identification apparatus 22 inputs the large patches, the medium patches, and the small patches into the lesion identification model and calculates a score for each patch. Next, the image identification apparatus 22 performs score interpolation to integrate scores between patches of different sizes. In this score interpolation, nearest neighbor interpolation is used for example. In this case, a large patch is divided into the size of the small patches, and the score of the original large patch is assigned to each of the divided patches. In the same way, a medium patch is divided into the size of the small patches, and the score of the original medium patch is assigned to each of the divided patches.

Note that score interpolation is not limited to nearest neighbor interpolation, and other methods such as a bilinear interpolation and a bicubic interpolation may be used. Although score interpolation is performed in units of small patches in the above example, score interpolation may be performed in units of pixels or voxels, for example. In the case of pixel units or voxel units in particular, it is preferable to perform score interpolation using bilinear interpolation or bicubic interpolation.

Next, the image identification apparatus 22 integrates the scores of the patches at the same position using Equation (1). As one example, in the integration of the patches P21a to P21c, the median value "0.1" is calculated as the score after integration. In addition, in the integration of the patches P22a to P22c in which the tumor is captured, the median value "0.7" is calculated as the score after integration. When the integrated score exceeds the threshold "0.5", the image identification apparatus 22 identifies that patch as a tumor. In the example in FIG. 9, only the patch P22 including a tumor is identified as a tumor, and the other patches are identified as normal. Note that in the example in FIG. 9, both the score of the patch P22b based on a medium patch and the score of the patch P22c which is a small patch exceed the threshold "0.5".

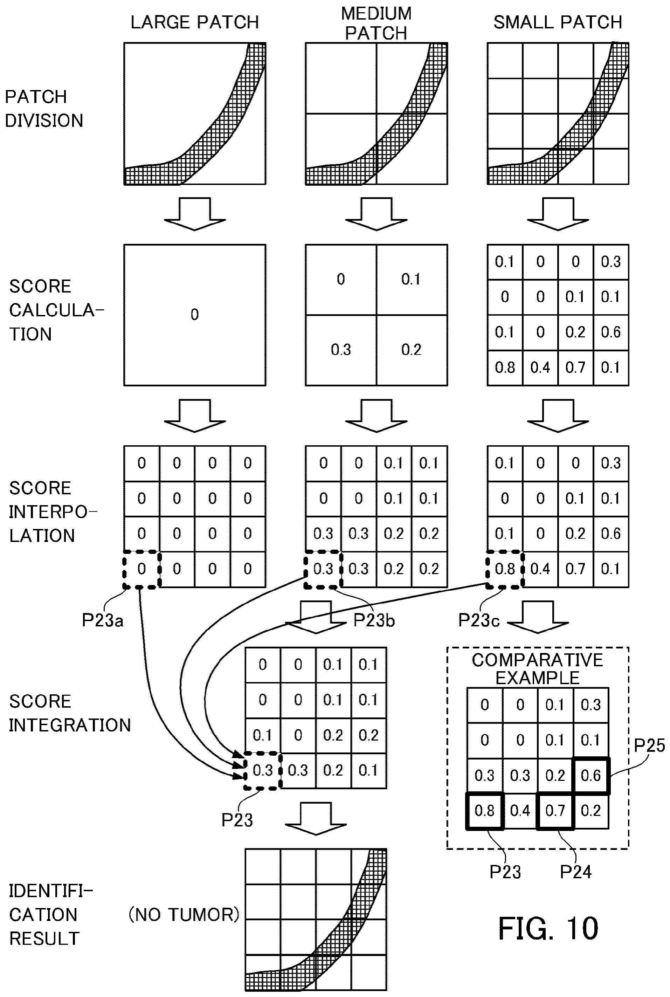

FIG. 10 is a second diagram depicting one example of the score integration process. FIG. 10 depicts a score integration process for an image region in which a blood vessel appears. Like FIG. 9, it is assumed that the score takes a value from 0 to 1 and the threshold for determining a tumor is 0.5.

Like the case depicted in FIG. 9, the image identification apparatus 22 divides the tomographic image into large patches, medium patches, and small patches, inputs each of the divided patches into the lesion identification model, and calculates a score for each patch. Next, the image identification apparatus 22 executes score interpolation to calculate the scores of the regions in units of small patches, and integrates the scores of patches at the same position using Equation (1). When the integrated score exceeds a threshold, the image identification apparatus 22 identifies the patch as a tumor.

In the example of FIG. 10, every patch is identified as normal. As one example, in the integration of the patches P23a to P23c, the median value "0.3" is calculated as the score after integration. Since this score is equal to or less than the threshold, the patch P23 corresponding to the patches P23a to P23c is identified as normal.

Here, in FIG. 10, an example where the scores have been integrated using the highest value as depicted in FIG. 6 is also depicted as a comparative example. In this comparative example, since the score after integration for the patch P23 is "0.8" and exceeds the threshold, the patch P23 is erroneously identified as a tumor. In addition, in this comparative example, the integrated scores of the patches P24 and P25 also exceed the threshold, so that the patches P24 and P25 are also erroneously identified as a tumor. In contrast, with the score integration process according to the present embodiment, it is possible to suppress the occurrence of such erroneous identification.

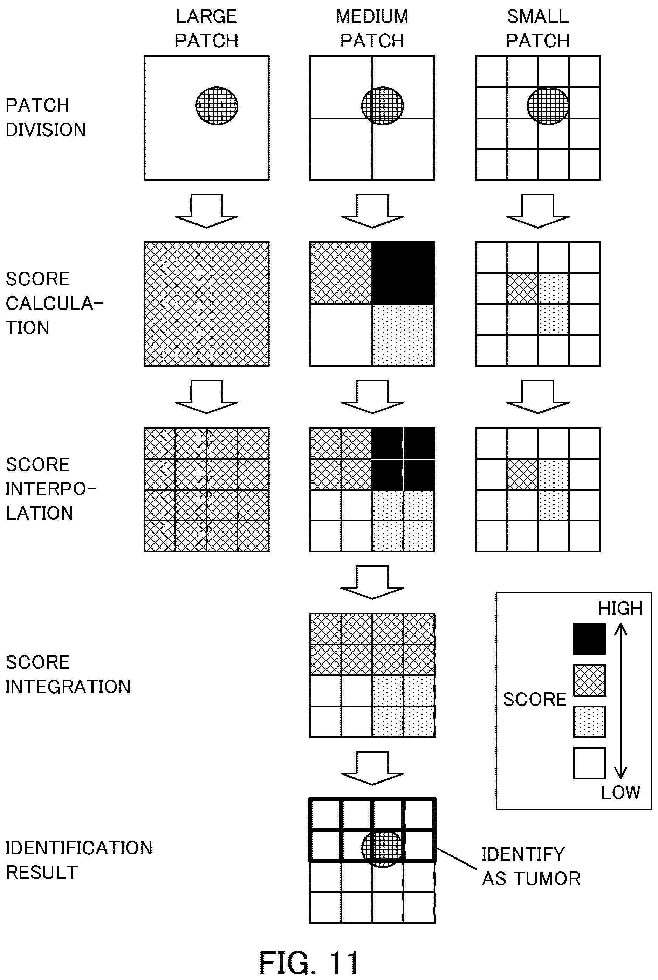

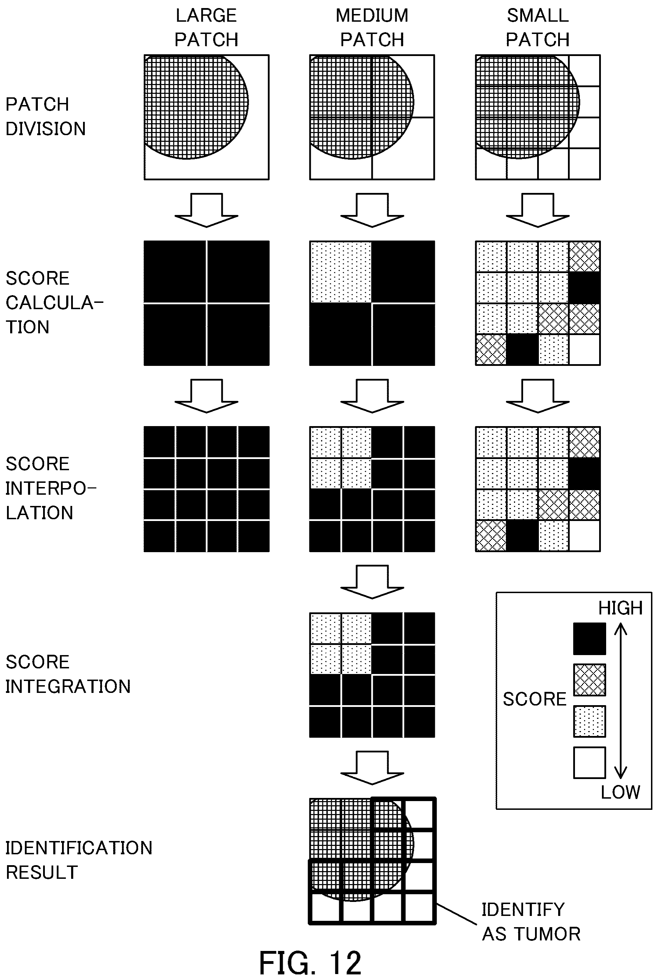

FIG. 11 is a third diagram depicting one example of the score integration process. FIG. 12 is a fourth diagram depicting one example of the score integration process.

FIG. 11 depicts one example of the score integration process for an image region in which a tumor appears larger than in the example in FIG. 9. FIG. 12 depicts one example of the score integration process for an image region in which a tumor appears even larger than in the example in FIG. 11. Note that in FIGS. 11 and 12, the scores are indicated on four levels, and the two higher levels are determined as a tumor by determination using a threshold. As can be understood from FIGS. 11 and 12, it is possible to detect a tumor even when the tumor appears larger.

Next, the processing of the training processing apparatus 12 and the image identification apparatus 22 will be described in more detail.

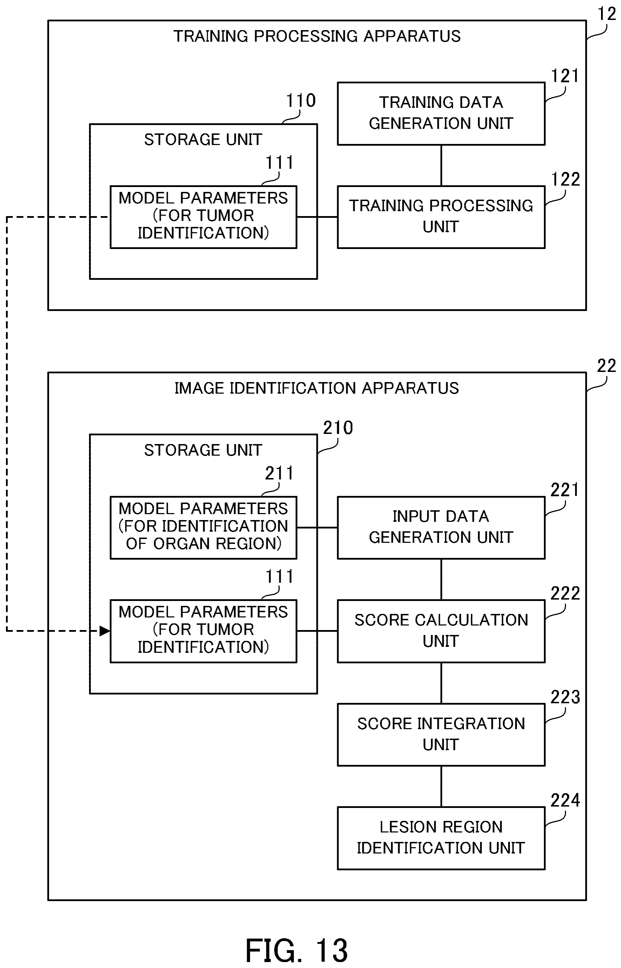

FIG. 13 depicts an example configuration of processing functions included in the training processing apparatus and the image identification apparatus.

The training processing apparatus 12 includes a storage unit 110, a training data generation unit 121, and a training processing unit 122.

The storage unit 110 is a storage area that is reserved in a storage apparatus included in the training processing apparatus 12. The storage unit 110 stores model parameters 111 indicating a model that has been trained for tumor identification (or "tumor identification model"). As one example, when the tumor identification model is formed by a neural network, the model parameters 111 include weighting coefficients between nodes on the neural network.

As one example, the processing of the training data generation unit 121 and the training processing unit 122 are realized by a processor included in the training processing apparatus 12 executing a predetermined program. The training data generation unit 121 generates training data for generating a tumor identification model by machine learning based on one or more tomographic image sets obtained by imaging by the MRI apparatus 11. The training processing unit 122 generates a tumor identification model by executing machine learning using the generated training data and stores the model parameters 111 indicating the tumor identification model in the storage unit 110.

The image identification apparatus 22 includes a storage unit 210, an input data generation unit 221, a score calculation unit 222, a score integration unit 223, and a lesion region identification unit 224.

The storage unit 210 is a storage area that is reserved in a storage device included in the image identification apparatus 22, such as the RAM 202 or the HDD 203. The storage unit 210 stores model parameters 211 indicating a model that has been trained for organ region identification (or "organ region identification model") and the model parameters 111 indicating the tumor identification model.

The organ region identification model is a trained model indicating an identifier device for identifying a region of an organ (here, the liver) in a tomographic image set, and is generated in advance by machine learning. The model parameters 111 are generated by the training processing apparatus 12, are read into the image identification apparatus 22 via a network or a portable recording medium for example, and are stored in the storage unit 210.

As one example, the processing of the input data generation unit 221, the score calculation unit 222, the score integration unit 223, and the lesion region identification unit 224 is realized by the processor 201 executing a predetermined program.

The input data generation unit 221 generates three-dimensional volume data based on a tomographic image set obtained by imaging by the MRI apparatus 21. The input data generation unit 221 inputs the volume data into the trained model based on the model parameters 211 and specifies voxels of an organ region out of voxels in the volume data. The input data generation unit 221 sets a plurality of slice planes in the volume data, generates a tomographic image for each slice plane, and generates patches of a plurality of sizes (three sizes in the present embodiment) from each tomographic image. However, only patches including the organ region are selected to be subjected to lesion identification.

The score calculation unit 222 inputs patches that have been generated for the lesion identification model based on the model parameters 111, and calculates a score indicating the probability of a tumor for each patch. In this score calculation, each input patch is enlarged or reduced as needed to a patch size defined for the lesion identification model (that is, the size of the patches that were used during training) and then inputted into the lesion identification model.

The score integration unit 223 integrates scores between patches of different sizes. In this integration process, large patches and medium patches are divided into the same size as the small patches by a score interpolation process where the score of the original patch is assigned to the divided patches. The scores are then integrated in units of small patch regions.

The lesion region identification unit 224 compares the integrated scores with a predetermined threshold and identifies each region where the score exceeds the threshold as a lesion (tumor) region. The lesion region identification unit 224 generates a display image indicating the lesion identification result and displays the display image on the display apparatus 204a.

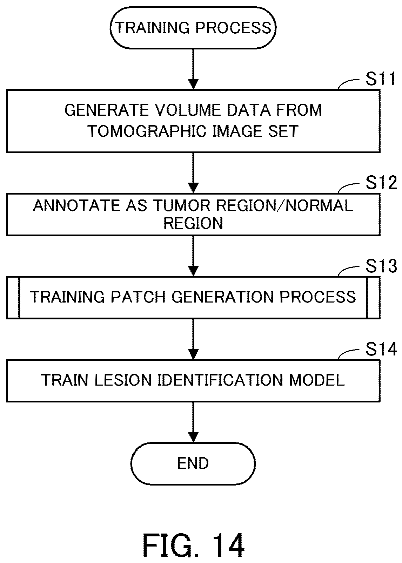

FIG. 14 is an example flowchart depicting a procedure of a training process by the training processing apparatus.

[Step S11] The training data generation unit 121 acquires a tomographic image set from the MRI apparatus 11. The training data generation unit 121 generates three-dimensional volume data based on the acquired tomographic image set.

[Step S12] The training data generation unit 121 annotates the respective voxels in the generated volume data as tumor regions and normal regions.

[Step S13] The training data generation unit 121 generates training patches based on the annotated volume data. As the training patches, tumor patches to which a tumor label has been assigned and normal patches to which a normal label has been assigned are generated.

[Step S14] The training processing unit 122 executes machine learning using the generated training patches, and generates a lesion identification model for identifying whether an input patch is a tumor region or a normal region. As one example, the training processing unit 122 generates a lesion identification model by deep learning using a neural network. The training processing unit 122 stores the model parameters 111 indicating the generated lesion identification model in the storage unit 110.

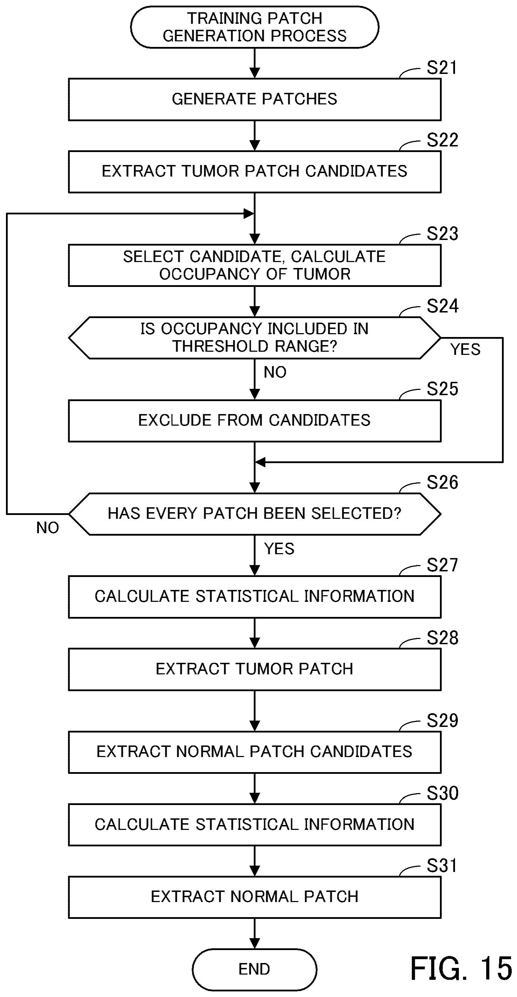

FIG. 15 is an example flowchart depicting the procedure of a training patch generation process.

[Step S21] The training data generation unit 121 regenerates a plurality of tomographic images for example by slicing the volume data at the same intervals as the vertical and horizontal sizes of the patches, and divides each tomographic image into patches. The size of the patches is the same as the size of the images inputted into the lesion identification model.

[Step S22] The training data generation unit 121 extracts patches including a tumor in the organ region (liver region) from the generated patches as tumor patch candidates.

[Step S23] The training data generation unit 121 selects one tumor patch candidate and calculates, based on an annotation result performed in voxel units, the occupancy of the area of the tumor in the image region of the tumor patch candidate.

[Step S24] The training data generation unit 121 determines whether the calculated occupancy is included in a range from the lower limit threshold $TH_{min}$ to the upper limit threshold $TH_{max}$ described earlier. When the occupancy is included in this threshold range, the processing proceeds to step S26, and when the occupancy is not included in this threshold range, the processing proceeds to step S25. Note that as one example, the lower limit threshold $TH_{min}$ and the upper limit threshold $TH_{max}$ are respectively calculated using Equations (5a) and (5b) described above.

[Step S25] The training data generation unit 121 excludes the tumor patch candidate so that the tumor patch candidate is not extracted as a tumor patch.

[Step S26] The training data generation unit 121 determines whether every tumor patch candidate has been selected. When there are unselected tumor patch candidates, the processing proceeds to step S23 to select one of the unselected tumor patch candidates. On the other hand, when every tumor patch candidate has been selected, the processing proceeds to step S27.

[Step S27] The training data generation unit 121 calculates an average value of luminance and a standard deviation of luminance as statistical information for each of the currently remaining tumor patch candidates.

[Step S28] The training data generation unit 121 extracts a predetermined number (which is two or more) of tumor patches out of the tumor patch candidates so that the average value of the luminance and the standard deviation of the luminance vary.

In MR images, a tumor region appears darker than surrounding regions inside an organ. This means that by extracting tumor patches with respectively different luminance distributions using the procedure described above, tumor patches including tumors of various forms, such as tumors of different sizes and tumors with different internal gradations, will be extracted. As a result, it becomes possible to generate a lesion identification model capable of correctly identifying tumor regions as tumors.

[Step S29] The training data generation unit 121 extracts, out of the generated patches, patches that do not include a tumor inside the organ region (liver region) as normal patch candidates.

[Step S30] The training data generation unit 121 calculates an average value of luminance and a standard deviation of luminance as statistical information for each of the normal patch candidates.

[Step S31] The training data generation unit 121 extracts a predetermined number (which is two or more) of tumor patches from the normal patch candidates so that the average value of the luminance and the standard deviation of the luminance are dispersed. By doing so, since normal patches where the patterns of the luminance distributions are respectively different are extracted, regions including a blood vessel and regions that do not include a blood vessel are extracted with a favorable balance. As a result, it becomes possible to generate a lesion identification model capable of correctly identifying regions including blood vessels as normal.

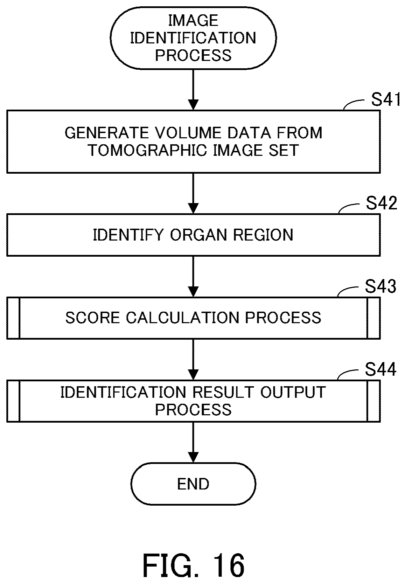

FIG. 16 is an example flowchart depicting the procedure of an image identification process performed by the image identification apparatus.

[Step S41] The input data generation unit 221 acquires a tomographic image set from the MRI apparatus 21 and generates three-dimensional volume data based on the acquired tomographic image set.

[Step S42] The input data generation unit 221 identifies an organ region (liver region) from the volume data using a model that has been trained for organ region identification and is formed of the model parameters 211.

This trained model is generated for example by deep learning using a large number of tomographic images as teacher images and teacher labels indicating whether each pixel in the tomographic images is an intra-organ region or an extra-organ region. In this case, in step S42, by inputting each tomographic image included in the tomographic image set into the trained model, it is determined whether each pixel in each tomographic image is an intra-organ region or an extra-organ region. After this, based on the determination result for each tomographic image, the voxels of an organ region are specified out of the voxels in the volume data.

[Step S43] A score calculation process is executed by the input data generation unit 221 and the score calculation unit 222. In this process, patches of different sizes are generated based on the volume data, and each patch is inputted into the lesion identification model so that a score indicating the probability of a tumor is calculated for each patch.

[Step S44] An identification result output process is executed by the score integration unit 223 and the lesion region identification unit 224. In this process, the score integration unit 223 integrates scores between patches of different sizes in units of small patch regions. The lesion region identification unit 224 compares the integrated score with a predetermined threshold, and identifies regions in which the score exceeds the threshold as a lesion (tumor) region. The lesion region identification unit 224 generates a display image indicating the lesion identification result and displays the display image on the display apparatus 204a.

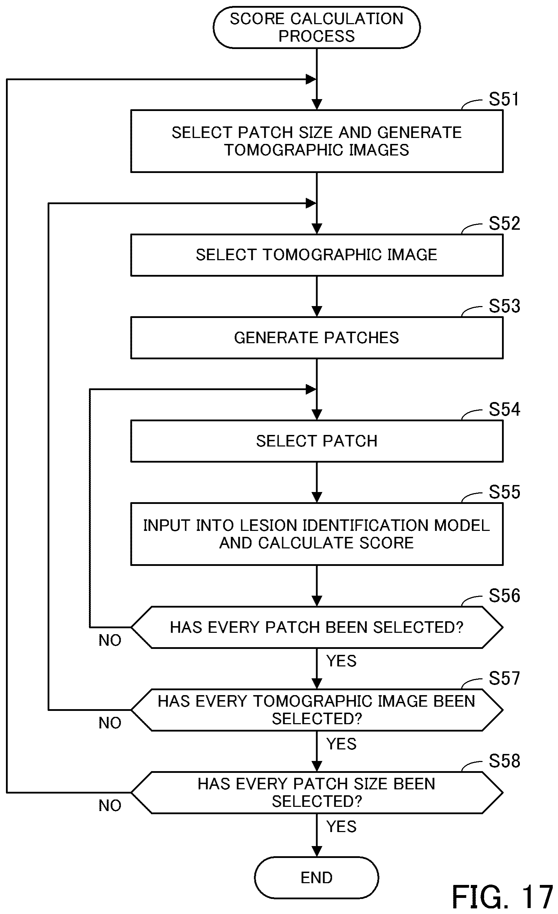

FIG. 17 is an example flowchart depicting the procedure of a score calculation process.

[Step S51] The input data generation unit 221 selects one patch size (scale). The input data generation unit 221 regenerates a plurality of tomographic images by slicing the volume data at the same intervals as the selected patch size (that is, the vertical and horizontal sizes of the patch).

[Step S52] The input data generation unit 221 selects one tomographic image.

[Step S53] The input data generation unit 221 divides the selected tomographic image by the patch size selected in step S51 to generate patches. However, only patches included in the organ region identified in step S42 of FIG. 16 are processed in the steps from step S54 onwards.

[Step S54] The score calculation unit 222 selects one of the generated patches.

[Step S55] The score calculation unit 222 inputs the selected patch into the lesion identification model formed by the model parameters 111, and calculates a score indicating the probability of a tumor.

In step S55, when the selected patch size does not match a defined patch size defined for the lesion identification model, the score calculation unit 222 enlarges or reduces the selected patch in accordance with the defined patch size and then inputs the enlarged or reduced patch into the lesion identification model. As one example, assume that the size of a large patch out the small patches, the medium patches, and the large patches matches the specified patch size. In this case, small patches and medium patches are enlarged to the size of a large patch, and the enlarged patches are inputted into the lesion identification model. On the other hand, large patches are directly inputted into the lesion identification model.

[Step S56] The score calculation unit 222 determines whether every patch has been selected. When there are unselected patches, the processing proceeds to step S54 to select one of the unselected patches. On the other hand, when every patch has been selected, the processing proceeds to step S57.

[Step S57] The score calculation unit 222 determines whether every tomographic image has been selected. When there are unselected tomographic images, the processing proceeds to step S52 to select one of the unselected tomographic images. On the other hand, when every tomographic image has been selected, the processing proceeds to step S58.

[Step S58] The score calculation unit 222 determines whether every patch size has been selected. When there is an unselected patch size, the processing proceeds to step S51 and one of the unselected patch sizes is selected. On the other hand, when every patch size has been selected, the processing ends.

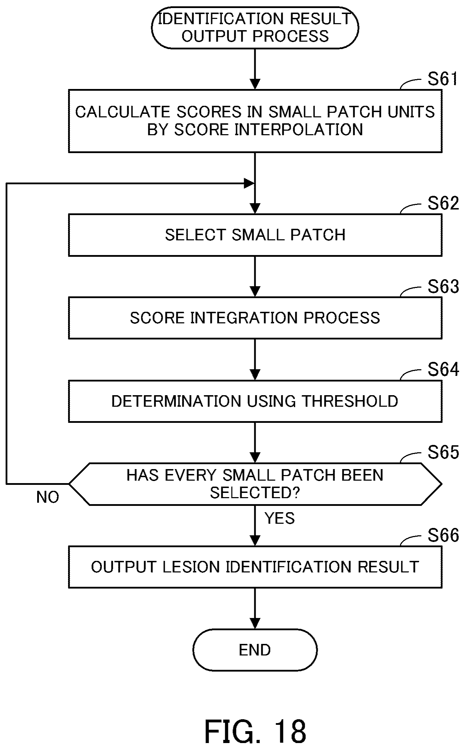

FIG. 18 is an example flowchart depicting the procedure of an identification result output process.

[Step S61] For large patches and medium patches, the score integration unit 223 calculates scores in small patch units by score interpolation. In more detail, the score integration unit 223 divides the large patch and the medium patch into small patch sizes, and assigns the scores of the original patches to the divided regions.

[Step S62] The score integration unit 223 selects one small patch.

[Step S63] The score integration unit 223 acquires a scores calculated from the selected small patch and a region at the same position as the selected small patch out of the regions divided from the large patch and the medium patch in step S61. The score integration unit 223 integrates the scores using any one of Equations (1) to (4) described earlier.

[Step S64] The lesion region identification unit 224 compares the integrated score with a predetermined threshold. The lesion region identification unit 224 identifies the region corresponding to the small patch as a lesion (tumor) area when the score exceeds a threshold, and identifies the region corresponding to the small patch as a normal region when the score is equal to or less than the threshold.

[Step S65] The lesion region identification unit 224 determines whether every small patch has been selected. When there are unselected small patches, the processing proceeds to step S62 to select one of the unselected small patches. On the other hand, when every small patch has been selected, the processing proceeds to step S66.

[Step S66] The lesion region identification unit 224 generates a display image indicating the lesion identification result and displays the display image on the display apparatus 204a.

According to the second embodiment described above, it is possible, regardless of the tumor size, to increase the likelihood of a tumor being detected without being missed, and possible to reduce the likelihood of a blood vessel being erroneously identified as a tumor. Accordingly, the identification accuracy of tumors is improved.


[Third Embodiment]

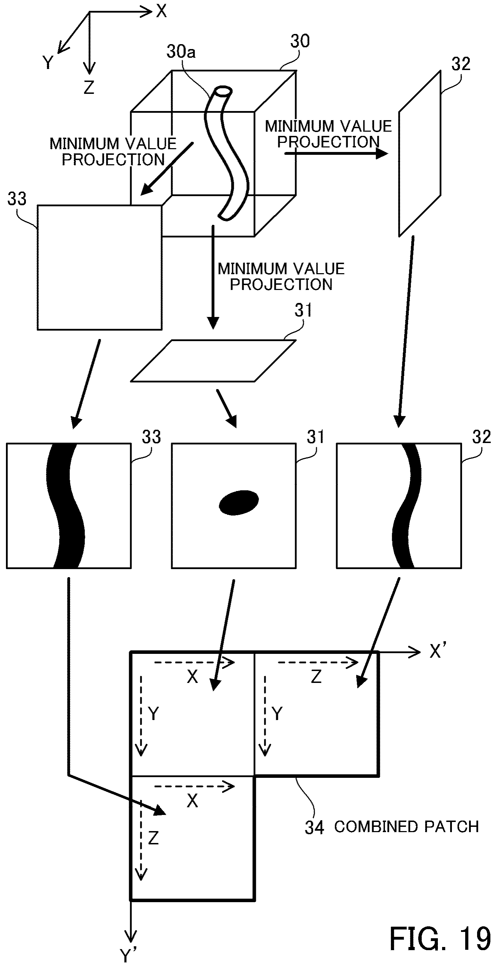

In the second embodiment described above, patches cut out from a tomographic image are directly inputted into the lesion identification model to calculate a score. In contrast, in this third embodiment, a combined patch like that depicted in FIG. 19 is generated and this combined patch is inputted into the lesion identification model. By doing so, it is possible to further reduce the likelihood of a blood vessel being erroneously identified as a tumor.

FIG. 19 is a first diagram useful in explaining a combined patch generation process. Note that in the following description, a direction from right to left for a human body in a standing state is defined as the X axis, a direction from back to front is defined as the Y axis, and a direction from top to bottom is defined as the Z axis. In this case, a tomographic image of an axial plane is an image across an X-Y plane, a tomographic image of a sagittal plane is an image across a Y-Z plane, and a tomographic image of a coronal plane is an image across a X-Z plane.

In the present embodiment, as input data for the lesion identification model, a minimum intensity projection image in the three axial directions is used based on a three-dimensional block cut out from volume data with sides that are equal to the vertical and horizontal sizes of a patch. As depicted in FIG. 19, a minimum intensity projection image 31 in the Z-axis direction is generated by performing minimum intensity projection in the Z-axis direction on each pixel on an X-Y plane (axial plane) in the block 30. A minimum intensity projection image 32 in the X-axis direction is generated by performing minimum intensity projection in the X-axis direction on each pixel on a Y-Z plane (sagittal plane) in the block 30. In addition, a minimum intensity projection image 33 in the Y-axis direction is generated by performing minimum intensity projection in the Y-axis direction on each pixel on an X-Z plane (coronal plane) in the block 30.

Note that as one example, pixel values of the minimum intensity projection image 31 in the Z-axis direction are obtained by the following calculation. Here, it is assumed that n tomographic images (here, tomographic images of an axial plane) are generated from a block, and the pixel value of the coordinates $(x, y)$ in the $i^{th}$ tomographic image is $g_i(x,y)$. In this case, the pixel value $h(x,y)$ at the coordinates $(x, y)$ in the minimum intensity projection image 31 is calculated using Equation (7), where "$\min(\cdots)$" indicates the minimum value out of the values included in $(\cdots)$.

$$h(x,y) = \min(g_i(x,y)), \quad i = 1,2, \cdots,n \quad \cdots(7)$$

Note that it is desirable to determine the value of n so that a tumor of a size for which detection is desirable is included in the block cut out from the volume data. For example, when the minimum size of a tumor to be detected is expressed as r, the distance between tomographic images is d, and it is possible to detect a tumor larger than half the patch size (the size of one side of a block), n is determined so as to satisfy n*d/2<r.

As one example, although a blood vessel 30a depicted in FIG. 19 extends in a curved manner, the blood vessel will be depicted with an elongated shape in at least one of the minimum intensity projection images 31 to 33 regardless of the actual shape of the blood vessel. In the example in FIG. 19, although the blood vessel 30a is depicted with an elliptical shape in the minimum intensity projection image 31, the blood vessel 30a is depicted in an elongated shape in the minimum intensity projection images 32 and 33. Accordingly, by using minimum intensity projection images in three directions as input data, the likelihood of a blood vessel being erroneously identified as a tumor is reduced, so that the identification accuracy of the lesion identification model is improved.

Also, in the present embodiment, the minimum intensity projection images in three directions generated based on a block are combined as a combined patch 34, which is a single image. Note that to avoid confusion in FIG. 19, the X axis and the Y axis in the combined patch 34 have been respectively indicated as the "X' axis" and "Y' axis".

As depicted in FIG. 19, in the combined patch 34, the minimum intensity projection image 31 for the Z-axis direction and the minimum intensity projection image 32 for the X-axis direction are adjacently combined in a state where their respective Y coordinates have been aligned. In addition, the minimum intensity projection image 31 for the Z-axis direction and the minimum intensity projection image 33 for the Y-axis direction are adjacently combined in a state where their respective X coordinates have been aligned.

In this case, the X coordinates and Y coordinates of the minimum intensity projection image 31 are used without amendment as the X' coordinates and Y' coordinates in the combined patch 34, respectively. The Y coordinates in the minimum intensity projection image 32 are used without amendment as the Y' coordinates in the combined patch 34, and values obtained by adding the patch size to the Z coordinates in the minimum intensity projection image 32 are used as the X' coordinates in the combined patch 34. In addition, the X coordinates in the minimum intensity projection image 33 are used without amendment as the X' coordinates in the combined patch 34, and values obtained by adding the patch size to the Z coordinates in the minimum intensity projection image 33 are used as the Y' coordinates in the combined patch 34.

In this way, the combined patch, which is two-dimensional image data, is inputted into the lesion identification model. The training data used for training the lesion identification model is also a combined patch obtained by combining minimum intensity projection images in three directions generated from an original block. That is, a label indicating a tumor or normal is added to each combined patch used for training, and machine learning of the lesion identification model is executed using these combined patches.

Note that a minimum intensity projection image generated based on a block is not necessarily an image that has been projected in a direction along a coordinate axis. However, it is desirable for the minimum intensity projection images to be projected in directions that are perpendicular to each other. Two minimum intensity projection images projected in two directions that are perpendicular to each other may be generated based on a block. In this case also, in at least one of the minimum intensity projection images, a blood vessel in the block will be depicted in an elongated shape that extends from one side to the same side or another side of the image.

In addition, in a hepatocyte contrast phase using an EOB contrast agent, both a tumor and a blood vessel appear darker than a surrounding region inside the liver. For this reason, a combined patch is generated by combining minimum intensity projection images. However, depending on differences in imaging method and/or the lesion to be identified, a region may appear brighter than surrounding regions. In this case, a combined patch may be generated by combining maximum intensity projection images.

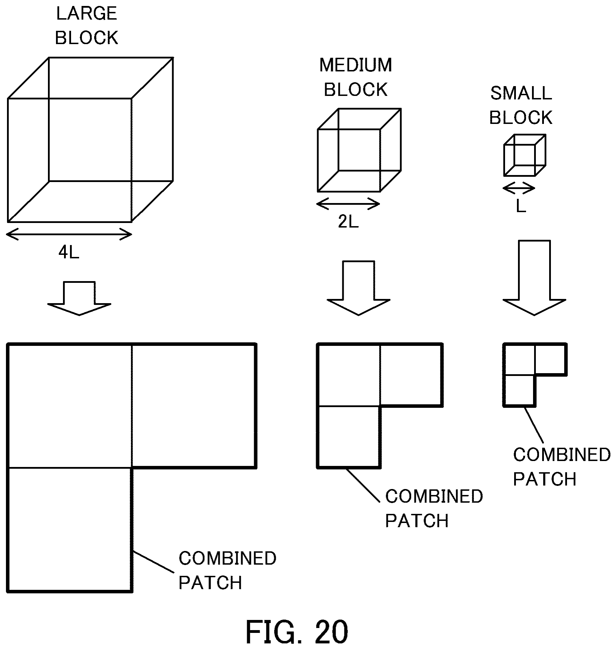

FIG. 20 is a second diagram useful in explaining a combined patch generation process. In the present embodiment, the input data generation unit 221 cuts out a large block, a medium block, and a small block from the volume data. When the length of one side of the small block is expressed as L, the length of one side of the medium block is 2L for example, and the length of one side of the large block is 4L for example.

The input data generation unit 221 generates a minimum intensity projection image in each of the three axial directions for each of the large block, the medium block, and the small block, and combines the generated minimum intensity projection images using the procedure depicted in FIG. 19 to generate a combined patch. As a result, combined patches corresponding to the large block, the medium block, and the small block are generated for the same position (the same voxel) in the volume data.

The score calculation unit 222 inputs the combined patches generated in this way into the lesion identification model, and calculates scores corresponding to the combined patches (that is, corresponding to the original block). The score integration unit 223 selects the respective combined patches generated based on the large block, the medium block, and the small block corresponding to the same voxel in the volume data, and integrates the scores calculated from the respective combined patches using any one of Equations (1) to (4) described above. The lesion region identification unit

**EP 4 651 074 A1**

224 compares the integrated score with a threshold to identify whether the voxel is a tumor or is normal.

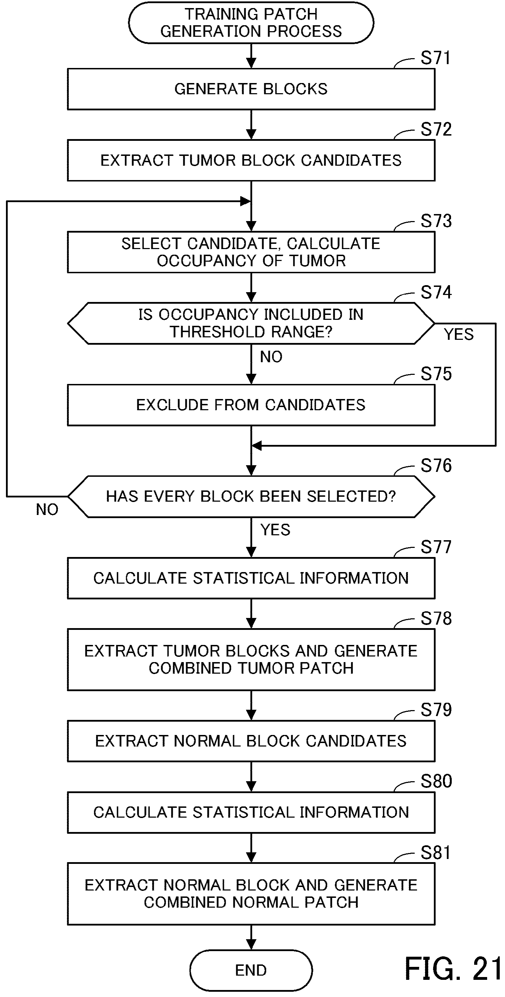

Next, a process of generating a training combined patch according to the third embodiment will be described. In the present embodiment, the processing in FIG. 21 described below is executed as the processing of step S13 in FIG. 14.

FIG. 21 is an example flowchart depicting the procedure of a training patch generation process.

[Step S71] The training data generation unit 121 divides the volume data into blocks.

[Step S72] The training data generation unit 121 extracts blocks including a tumor in the organ region (liver region) from the blocks as tumor block candidates.

[Step S73] The training data generation unit 121 selects one tumor block candidate and calculates, based on annotation results in units of voxels, the occupancy of the volume of a tumor in the three-dimensional region of the selected tumor block candidate.

[Step S74] The training data generation unit 121 determines whether the occupancy is included in the range from the lower limit threshold $TH_{min}$ to the upper limit threshold $TH_{max}$ described earlier. When the occupancy is within the threshold range, the processing proceeds to step S76. When the occupancy is not within the threshold range, the processing proceeds to step S75. Note that the lower limit threshold $TH_{min}$ and the upper limit threshold $TH_{max}$ are respectively calculated using Equations (6a) and (6b) described earlier, for example.

[Step S75] The training data generation unit 121 excludes the tumor block candidate so that the tumor block candidate is not extracted as a tumor block.

[Step S76] The training data generation unit 121 determines whether every tumor block candidate has been selected. When there are unselected tumor block candidates, the processing proceeds to step S73 to select one of the unselected tumor block candidates. On the other hand, when every tumor block candidate has been selected, the processing proceeds to step S77.

[Step S77] The training data generation unit 121 calculates an average value of luminance and a standard deviation of luminance as statistical information for each of the remaining tumor block candidates.

[Step S78] The training data generation unit 121 extracts a predetermined number (which is two or more) of tumor blocks from the tumor block candidates so that the average value of the luminance and the standard deviation of the luminance are dispersed. The training data generation unit 121 then generates a minimum intensity projection image for each of the three axial directions for each of the extracted tumor blocks, and combines the generated minimum intensity projection images to generate a combined tumor patch to which a tumor label is assigned.

[Step S79] The training data generation unit 121 extracts, from the generated blocks, a block that does not include a tumor in the organ region (liver region) as a normal block candidate.

[Step S80] The training data generation unit 121 calculates an average value of luminance and a standard deviation of luminance as statistical information for each of the normal block candidates.

[Step S81] The training data generation unit 121 extracts a predetermined number (which is two or more) of normal blocks from the normal block candidates so that the average value of the luminance and the standard deviation of the luminance are dispersed. The training data generation unit 121 then generates a minimum intensity projection image for each of the three axial directions for each of the extracted normal blocks, and combines the generated minimum intensity projection images to generate a combined normal patch to which a normal label is assigned.

When training combined patches have been generated in this way, the training processing unit 122 executes machine learning using the generated training combined patches to generate a lesion identification model for identifying whether an inputted combined patch is a tumor region or a normal region. The training processing unit 122 stores the model parameters 111 indicating the generated lesion identification model in the storage unit 110.

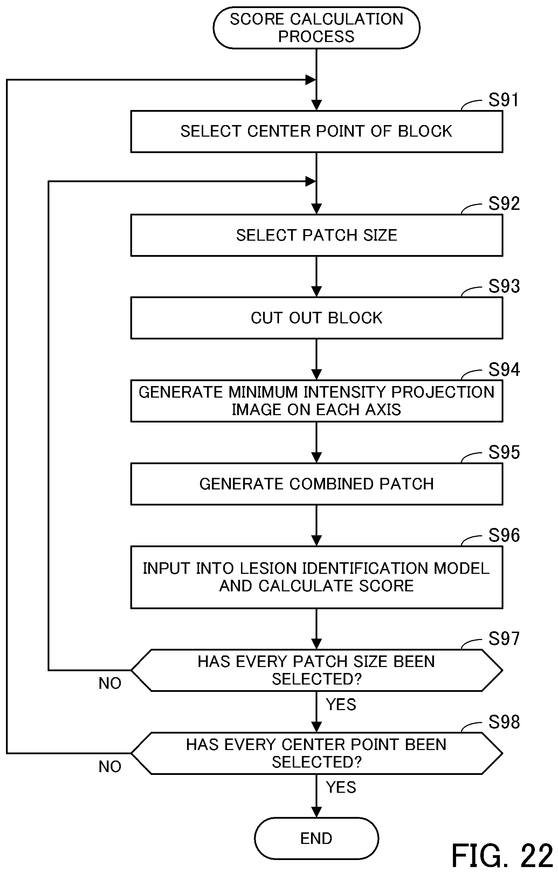

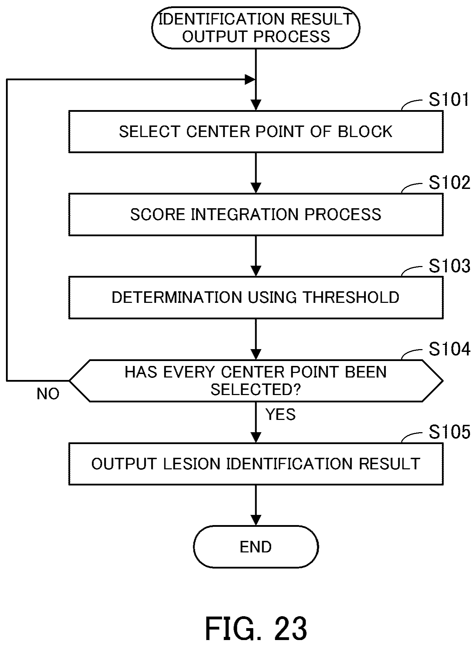

Next, an image identification process according to the third embodiment will be described. In the present embodiment, the processing in FIG. 22 is executed as the processing of step S43 in FIG. 16, and the processing in FIG. 23 is executed as the processing of step S44 in FIG. 16.

FIG. 22 is an example flowchart depicting the procedure of a score calculation process.

[Step S91] The input data generation unit 221 selects a center point of a block out of the voxels in the organ region. The center point of the block is a point that is at least the center of a small block, and is generated for example by shifting a three-dimensional window with the same size as one side of a small block in units of a predetermined distance (for example, half of the patch size).

[Step S92] The input data generation unit 221 selects one patch size (scale).

[Step S93] The input data generation unit 221 cuts out, from the volume data, a block whose center is the selected center point and whose sides match the selected patch size in length.

[Step S94] The input data generation unit 221 generates a minimum intensity projection image for each of the three axial directions based on the block that has been cut out.

[Step S95] The input data generation unit 221 combines the generated minimum intensity projection images using the method depicted in FIG. 19 to generate a combined patch.

[Step S96] The score calculation unit 222 inputs the selected combined patch into the lesion identification model formed by the model parameters 111, and calculates a score indicating the probability of a tumor. In step S96, when the selected patch size does not match a patch size that is defined for the lesion identification model (that is, the size of the block or patch from which the training combined patches were generated), the score calculation unit 222 enlarges or reduces the selected combined patch in accordance with the defined patch size and then inputs the enlarged or reduced combined patch into the lesion identification model.

[Step S97] The score calculation unit 222 determines whether every patch size has been selected. When there are unselected patch sizes, the processing proceeds to step S92 to select one of the unselected patch sizes. On the other hand, when every patch size has been selected, the processing proceeds to step S98. In the latter case, the score is calculated from the combined patches generated based on the large block, the medium block, and the small block centered on the selected center point.

[Step S98] The score calculation unit 222 determines whether the center point of every block has been selected. When there are unselected center points, the processing proceeds to step S91 to select one of the unselected center points. On the other hand, when every center point has been selected, the processing ends.

FIG. 23 is an example flowchart depicting the procedure of an identification result output process.

[Step S101] The score integration unit 223 selects the center point of a block out of the voxels in the organ region.

[Step S102] The score integration unit 223 specifies each combined patch generated based on each of the large block, the medium block, and the small block centered on the selected center point, and acquires a score calculated from each specified combined patch. The score integration unit 223 integrates the scores using any one of Equations (1) to (4) described earlier.

[Step S103] The lesion region identification unit 224 compares the integrated score with a predetermined threshold. The lesion region identification unit 224 identifies the position of the selected center point as a lesion (tumor) area when the score exceeds a threshold, and identifies the position of the selected center point as a normal area when the score is equal to or less than the threshold.

[Step S104] The lesion region identification unit 224 determines whether every center point has been selected. When there are unselected center points, the processing proceeds to step S101 to select one of the unselected center points. On the other hand, when every center point has been selected, the processing proceeds to step S105.

[Step S105] The lesion region identification unit 224 generates a display image indicating the lesion identification result and displays the display image on the display apparatus 204a.

According to the third embodiment described above, compared to the second embodiment, it is possible to more accurately distinguish between a tumor and a normal state (in particular, blood vessels).

Note that the processing functions of the devices (for example, the image identification apparatus 1, the training processing apparatus 12, and the image identification apparatus 22) described in the above embodiments may be implemented by a computer. When doing so, a program in which the processing contents of every function of the devices are written is provided, and the processing functions are realized on a computer by executing the program on the computer. The program in which such processing contents are written may be recorded on a computer-readable recording medium. Examples of computer-readable recording media include a magnetic storage device, an optical disc, and a semiconductor memory. Examples of magnetic storage devices include a hard disk drive (HDD) and magnetic tape. Examples of optical discs include a compact disc (CD), a digital versatile disc (DVD), and a Blu-ray disc (BD, registered trademark).

When the program is distributed, as one example a portable recording medium such as a DVD or a CD on which the program has been recorded is sold. Alternatively, the program may be stored in a storage device of a server computer and transferred from the server computer to another computer via a network.

As one example, the computer that executes the program may store the program recorded on the portable recording medium or the program transferred from the server computer in its own storage device. The computer may then read the program from its own storage device and execute processing according to the program. The computer may also read the program directly from the portable recording medium and execute processing according to the program. In addition, whenever a program is transferred from a server computer connected via a network, the computer may sequentially execute processing in accordance with the received program.

Reference Signs List

| | |
|---|---|
| 1 | Image identification apparatus |
| 1a | Processing unit |
| 2 | Tomographic image |
| 2a | Position |
| 3a to 3c | Partial image |

Claims

1. An image identification method executed by a computer, the image identification method comprising:

cutting out, from a tomographic image obtained by imaging of an inside of a human body, a plurality of partial images that include a same position in the tomographic image and have different sizes;
calculating a probability of each of the plurality of partial images being a region with a specified lesion;
calculating an integrated value by integrating the probabilities calculated from each of the plurality of partial images using a calculation that increases a contribution of probabilities calculated from partial images corresponding to at least an intermediate size out of the plurality of partial images; and
identifying the same position as a region with the specified lesion upon determining that the integrated value exceeds a predetermined threshold.

2. The image identification method according to claim 1,

wherein the calculating of the probability is executed by using a trained model for identifying whether an input image is a region of the specified lesion, and
wherein the trained model is generated by machine learning using images in which a ratio of an area of a region with the specified lesion in the images is equal to or greater than a predetermined lower threshold, which is greater than 0, and equal to or less than a predetermined upper threshold, which is less than 1, out of images in which the specified lesion appears as training images to which a label indicating regions of the specified lesion is assigned.

3. The image identification method according to claim 1,
wherein the integrated value is calculated as a median value of the probabilities calculated from the plurality of partial images.

4. The image identification method according to claim 1,
wherein the integrated value is calculated by a calculation that increases a contribution of the probability calculated from a partial image of an intermediate first size out of the plurality of partial images and either the probability calculated from a partial image of a second size that is larger than the first size or the probability calculated from a partial image of a third size that is smaller than the first size, whichever is larger than another.

5. An image identification method executed by a computer, the image identification method comprising:

cutting out, from three-dimensional volume data generated based on a plurality of tomographic images obtained by imaging of an inside of a human body, a plurality of three-dimensional partial regions that include a same position in the volume data and have different sizes;
generating, for each three-dimensional partial region of the plurality of three-dimensional partial regions, a plurality of projection images by performing minimum intensity projection or maximum intensity projection of values of voxels in said each three-dimensional partial region in a plurality of directions that are perpendicular to each other, and calculating a probability of the same position being a region with a specified lesion based on the generated plurality of projection images;
calculating an integrated value by integrating the probabilities calculated from each of the plurality of three-dimensional partial regions using a calculation that increases a contribution of probabilities calculated from three-dimensional partial regions corresponding to at least an intermediate size out of the plurality of three-dimensional partial regions; and
identifying the same position as a region with the specified lesion upon determining that the integrated value exceeds a predetermined threshold.

6. The image identification method according to claim 5,

wherein the calculating of the probability is executed by using a trained model that receives an input of a plurality of input projection images generated by performing minimum intensity projection or maximum intensity projection of values of voxels of a three-dimensional image in the plurality of directions and identifies whether the three-dimensional image is a region with the specified lesion, and
the trained model is generated by a model generation process including:

cutting out a plurality of training partial regions, which are three-dimensional regions of a predetermined size, from three-dimensional training volume data based on a plurality of training tomographic images obtained by imaging of an inside of a human body;

specifying, from the plurality of training partial regions, a lesion partial region in which a ratio of a volume of a region of the specified lesion is equal to or larger than a predetermined lower threshold, which is larger than 0, and equal to or smaller than a predetermined upper threshold, which is smaller than 1; and

performing machine learning by using a plurality of training projection images generated by performing minimum intensity projection or maximum intensity projection of values of voxels of the specified lesion partial region in the plurality of directions as training data to which a label indicating regions of the specified lesion is assigned.

7. An image identification program that causes a computer to execute a process comprising:

cutting out, from a tomographic image obtained by imaging of an inside of a human body, a plurality of partial images that include a same position in the tomographic image and have different sizes;

calculating a probability of each of the plurality of partial images being a region with a specified lesion;

calculating an integrated value by integrating the probabilities calculated from each of the plurality of partial images using a calculation that increases a contribution of probabilities calculated from partial images corresponding to at least an intermediate size out of the plurality of partial images; and

identifying the same position as a region with the specified lesion upon determining that the integrated value exceeds a predetermined threshold.

8. The image identification program according to claim 7,

wherein the calculating of the probability is executed by using a trained model for identifying whether an input image is a region of the specified lesion, and

wherein the trained model is generated by machine learning using images in which a ratio of an area of a region with the specified lesion in the images is equal to or greater than a predetermined lower threshold, which is greater than 0, and equal to or less than a predetermined upper threshold, which is less than 1, out of images in which the specified lesion appears as training images to which a label indicating regions of the specified lesion is assigned.

9. The image identification program according to claim 7,
wherein the integrated value is calculated as a median value of the probabilities calculated from the plurality of partial images.

10. The image identification program according to claim 7,
wherein the integrated value is calculated by a calculation that increases a contribution of the probability calculated from a partial image of an intermediate first size out of the plurality of partial images and either the probability calculated from a partial image of a second size that is larger than the first size or the probability calculated from a partial image of a third size that is smaller than the first size, whichever is larger than another.

11. An image identification program that causes a computer to execute a process comprising:

cutting out, from three-dimensional volume data generated based on a plurality of tomographic images obtained by imaging of an inside of a human body, a plurality of three-dimensional partial regions that include a same position in the volume data and have different sizes;

generating, for each three-dimensional partial region of the plurality of three-dimensional partial regions, a plurality of projection images by performing minimum intensity projection or maximum intensity projection of values of voxels in said each three-dimensional partial region in a plurality of directions that are perpendicular to each other, and calculating a probability of the same position being a region with a specified lesion based on the generated plurality of projection images;

calculating an integrated value by integrating the probabilities calculated from each of the plurality of three-dimensional partial regions using a calculation that increases a contribution of probabilities calculated from three-dimensional partial regions corresponding to at least an intermediate size out of the plurality of three-dimensional partial regions; and

identifying the same position as a region with the specified lesion upon determining that the integrated value exceeds a predetermined threshold.

12. The image identification program according to claim 11,

wherein the calculating of the probability is executed by using a trained model that receives an input of a plurality of input projection images generated by performing minimum intensity projection or maximum intensity projection of values of voxels of a three-dimensional image in the plurality of directions and identifies whether the three-dimensional image is a region with the specified lesion, and
the trained model is generated by a model generation process including:

cutting out a plurality of training partial regions, which are three-dimensional regions of a predetermined size, from three-dimensional training volume data based on a plurality of training tomographic images obtained by imaging of an inside of a human body;
specifying, from the plurality of training partial regions, a lesion partial region in which a ratio of a volume of a region of the specified lesion is equal to or larger than a predetermined lower threshold, which is larger than 0, and equal to or smaller than a predetermined upper threshold, which is smaller than 1; and
performing machine learning by using a plurality of training projection images generated by performing minimum intensity projection or maximum intensity projection of values of voxels of the specified lesion partial region in the plurality of directions as training data to which a label indicating regions of the specified lesion is assigned.

IMAGE IDENTIFICATION
APPARATUS                    1

TOMOGRAPHIC
IMAGE            →            PROCESSING        →   IDENTIFICATION
                             UNIT      1a            RESULT

TOMOGRAPHIC
IMAGE

2a                                    2

PARTIAL
IMAGES            3a              3b           3c

PROBABILITY
= 0.8

PROBABILITY
= 0.3

PROBABILITY
= 0

INTEGRATION OF
PROBABILITIES

INTEGRATED VALUE = 0.3

IDENTIFICATION USING
THRESHOLD

INTEGRATED VALUE ≤ 0.5
→ IDENTIFY AS "NO LESION"

# FIG. 1

FIG. 2

FIG. 3

S11

ACQUIRE TOMOGRAPHIC IMAGE SET

S12

GENERATE PATCHES

S13

IDENTIFY LESION USING
IDENTIFICATION MODEL

LESION A     LESION B     NORMAL

FIG. 4

FIG. 5

FIG. 6

FIG. 7

OCCUPANCY $>$ UPPER
THRESHOLD
$\rightarrow$ EXCLUDE

OCCUPANCY $<$ LOWER
THRESHOLD
$\rightarrow$ EXCLUDE

FIG. 8

FIG. 9

|  | LARGE PATCH | MEDIUM PATCH | SMALL PATCH |
|---|---|---|---|

PATCH DIVISION

SCORE CALCULA-TION

LARGE PATCH: 0

MEDIUM PATCH:

| 0 | 0.1 |
|---|---|
| 0.3 | 0.2 |

SMALL PATCH:

| 0.1 | 0 | 0 | 0.3 |
|---|---|---|---|
| 0 | 0 | 0.1 | 0.1 |
| 0.1 | 0 | 0.2 | 0.6 |
| 0.8 | 0.4 | 0.7 | 0.1 |

SCORE INTERPO-LATION

| 0 | 0 | 0 | 0 |
|---|---|---|---|
| 0 | 0 | 0 | 0 |
| 0 | 0 | 0 | 0 |
| 0 | 0 | 0 | 0 |

| 0 | 0 | 0.1 | 0.1 |
|---|---|---|---|
| 0 | 0 | 0.1 | 0.1 |
| 0.3 | 0.3 | 0.2 | 0.2 |
| 0.3 | 0.3 | 0.2 | 0.2 |

| 0.1 | 0 | 0 | 0.3 |
|---|---|---|---|
| 0 | 0 | 0.1 | 0.1 |
| 0.1 | 0 | 0.2 | 0.6 |
| 0.8 | 0.4 | 0.7 | 0.1 |

P23a    P23b    P23c

SCORE INTEGRATION

| 0 | 0 | 0.1 | 0.1 |
|---|---|---|---|
| 0 | 0 | 0.1 | 0.1 |
| 0.1 | 0 | 0.2 | 0.2 |
| 0.3 | 0.3 | 0.2 | 0.1 |

P23

COMPARATIVE EXAMPLE

| 0 | 0 | 0.1 | 0.3 |
|---|---|---|---|
| 0 | 0 | 0.1 | 0.1 |
| 0.3 | 0.3 | 0.2 | 0.6 |
| 0.8 | 0.4 | 0.7 | 0.2 |

P25

P23    P24

IDENTIFI-CATION RESULT

(NO TUMOR)

FIG. 10

FIG. 11

FIG. 12

TRAINING PROCESSING APPARATUS — 12

STORAGE UNIT — 110

MODEL PARAMETERS (FOR TUMOR IDENTIFICATION) — 111

TRAINING DATA GENERATION UNIT — 121

TRAINING PROCESSING UNIT — 122

IMAGE IDENTIFICATION APPARATUS — 22

STORAGE UNIT — 210

MODEL PARAMETERS (FOR IDENTIFICATION OF ORGAN REGION) — 211

MODEL PARAMETERS (FOR TUMOR IDENTIFICATION) — 111

INPUT DATA GENERATION UNIT — 221

SCORE CALCULATION UNIT — 222

SCORE INTEGRATION UNIT — 223

LESION REGION IDENTIFICATION UNIT — 224

FIG. 13

```
                    ( TRAINING PROCESS )
                            │
                            ▼                    ⌐ S11
        ┌───────────────────────────────────────┐
        │      GENERATE VOLUME DATA FROM         │
        │        TOMOGRAPHIC IMAGE SET           │
        └───────────────────────────────────────┘
                            │
                            ▼                    ⌐ S12
        ┌───────────────────────────────────────┐
        │   ANNOTATE AS TUMOR REGION/NORMAL      │
        │               REGION                   │
        └───────────────────────────────────────┘
                            │
                            ▼                    ⌐ S13
        ┌┃─────────────────────────────────────┃┐
        │┃  TRAINING PATCH GENERATION PROCESS   ┃│
        └┃─────────────────────────────────────┃┘
                            │
                            ▼                    ⌐ S14
        ┌───────────────────────────────────────┐
        │     TRAIN LESION IDENTIFICATION MODEL  │
        └───────────────────────────────────────┘
                            │
                            ▼
                       (    END    )
```

# FIG. 14

TRAINING PATCH
GENERATION PROCESS

S21

GENERATE PATCHES

S22

EXTRACT TUMOR PATCH CANDIDATES

S23

SELECT CANDIDATE, CALCULATE
OCCUPANCY OF TUMOR

S24

IS OCCUPANCY INCLUDED IN
THRESHOLD RANGE? — YES

NO

S25

EXCLUDE FROM CANDIDATES

S26

HAS EVERY PATCH BEEN SELECTED?

NO

YES

S27

CALCULATE STATISTICAL INFORMATION

S28

EXTRACT TUMOR PATCH

S29

EXTRACT NORMAL PATCH CANDIDATES

S30

CALCULATE STATISTICAL INFORMATION

S31

EXTRACT NORMAL PATCH

END

FIG. 15

IMAGE
IDENTIFICATION
PROCESS

S41

GENERATE VOLUME DATA FROM
TOMOGRAPHIC IMAGE SET

S42

IDENTIFY ORGAN REGION

S43

SCORE CALCULATION PROCESS

S44

IDENTIFICATION RESULT OUTPUT
PROCESS

END

FIG. 16

FIG. 17

```
        ┌─────────────────────────┐
        │ IDENTIFICATION RESULT   │
        │   OUTPUT PROCESS        │
        └─────────────────────────┘
                    │
                    ▼                         S61
        ┌─────────────────────────────────────┐
        │ CALCULATE SCORES IN SMALL PATCH UNITS│
        │      BY SCORE INTERPOLATION          │
        └─────────────────────────────────────┘
                    │
                    ▼                         S62
        ┌─────────────────────────────────────┐
        │         SELECT SMALL PATCH           │
        └─────────────────────────────────────┘
                    │
                    ▼                         S63
        ┌─────────────────────────────────────┐
        │      SCORE INTEGRATION PROCESS       │
        └─────────────────────────────────────┘
                    │
                    ▼                         S64
        ┌─────────────────────────────────────┐
        │     DETERMINATION USING THRESHOLD    │
        └─────────────────────────────────────┘
                    │
                    ▼                         S65
        ╱───────────────────────────────────────╲
    NO  │   HAS EVERY SMALL PATCH BEEN           │
        ╲            SELECTED?                   ╱
        ─────────────────────────────────────────
                    │ YES
                    ▼                         S66
        ┌─────────────────────────────────────┐
        │  OUTPUT LESION IDENTIFICATION RESULT │
        └─────────────────────────────────────┘
                    │
                    ▼
              ┌───────────┐
              │    END    │
              └───────────┘
```

FIG. 18

FIG. 19

LARGE
BLOCK

MEDIUM
BLOCK

SMALL
BLOCK

4L

2L

L

COMBINED
PATCH

COMBINED
PATCH

COMBINED
PATCH

FIG. 20

```
        ┌─────────────────────────┐
        │  TRAINING PATCH         │
        │  GENERATION PROCESS     │
        └─────────────────────────┘
                    │
                    ▼                    S71
        ┌─────────────────────────────┐
        │    GENERATE BLOCKS          │
        └─────────────────────────────┘
                    │
                    ▼                    S72
        ┌─────────────────────────────┐
        │ EXTRACT TUMOR BLOCK         │
        │ CANDIDATES                  │
        └─────────────────────────────┘
                    │
                    ▼                    S73
        ┌─────────────────────────────┐
        │ SELECT CANDIDATE, CALCULATE │
        │ OCCUPANCY OF TUMOR          │
        └─────────────────────────────┘
                    │
                    ▼                    S74
        ╱ IS OCCUPANCY INCLUDED IN ╲ ──── YES ──┐
        ╲ THRESHOLD RANGE?          ╱           │
                    │ NO                         │
                    ▼                    S75     │
        ┌─────────────────────────────┐         │
        │ EXCLUDE FROM CANDIDATES     │         │
        └─────────────────────────────┘         │
                    │ ◄───────────────────────────┘
                    ▼                    S76
        ╱ HAS EVERY BLOCK BEEN SELECTED? ╲ ── NO
        ╲                                ╱
                    │ YES
                    ▼                    S77
        ┌─────────────────────────────┐
        │ CALCULATE STATISTICAL       │
        │ INFORMATION                 │
        └─────────────────────────────┘
                    │
                    ▼                    S78
        ┌─────────────────────────────┐
        │ EXTRACT TUMOR BLOCKS AND    │
        │ GENERATE COMBINED TUMOR     │
        │ PATCH                       │
        └─────────────────────────────┘
                    │
                    ▼                    S79
        ┌─────────────────────────────┐
        │ EXTRACT NORMAL BLOCK        │
        │ CANDIDATES                  │
        └─────────────────────────────┘
                    │
                    ▼                    S80
        ┌─────────────────────────────┐
        │ CALCULATE STATISTICAL       │
        │ INFORMATION                 │
        └─────────────────────────────┘
                    │
                    ▼                    S81
        ┌─────────────────────────────┐
        │ EXTRACT NORMAL BLOCK AND    │
        │ GENERATE COMBINED NORMAL    │
        │ PATCH                       │
        └─────────────────────────────┘
                    │
                    ▼
              ┌───────────┐
              │    END    │
              └───────────┘
```

FIG. 21

SCORE CALCULATION
PROCESS

S91
SELECT CENTER POINT OF BLOCK

S92
SELECT PATCH SIZE

S93
CUT OUT BLOCK

S94
GENERATE MINIMUM INTENSITY PROJECTION
IMAGE ON EACH AXIS

S95
GENERATE COMBINED PATCH

S96
INPUT INTO LESION IDENTIFICATION MODEL
AND CALCULATE SCORE

S97
HAS EVERY PATCH SIZE BEEN
SELECTED?

NO    YES

S98
HAS EVERY CENTER POINT BEEN
SELECTED?

NO    YES

END

FIG. 22

```
        ╭──────────────────────────╮
        │  IDENTIFICATION RESULT    │
        │    OUTPUT PROCESS         │
        ╰──────────────────────────╯
                    │
      ┌─────────────┼─────────────────────────┐
      │             ▼                      ┌ S101
      │  ┌───────────────────────────────────┐
      │  │   SELECT CENTER POINT OF BLOCK    │
      │  └───────────────────────────────────┘
      │             │                      ┌ S102
      │             ▼
      │  ┌───────────────────────────────────┐
      │  │     SCORE INTEGRATION PROCESS     │
      │  └───────────────────────────────────┘
      │             │                      ┌ S103
      │             ▼
      │  ┌───────────────────────────────────┐
      │  │   DETERMINATION USING THRESHOLD   │
      │  └───────────────────────────────────┘
      │             │                      ┌ S104
      │             ▼
      │  ╱───────────────────────────────────╲
      └──│  HAS EVERY CENTER POINT BEEN       │
      NO ╲         SELECTED?                  ╱
         └───────────────────────────────────┘
                    │ YES
                    ▼                      ┌ S105
         ┌───────────────────────────────────┐
         │  OUTPUT LESION IDENTIFICATION RESULT │
         └───────────────────────────────────┘
                    │
                    ▼
              ╭──────────╮
              │   END    │
              ╰──────────╯
```

# FIG. 23

## INTERNATIONAL SEARCH REPORT

International application No.

**PCT/JP2023/044155**

| A. | CLASSIFICATION OF SUBJECT MATTER |
|---|---|

*G06T 7/00*(2017.01)i; *A61B 5/055*(2006.01)i
FI:   G06T7/00 612; G06T7/00 350B; G06T7/00 660Z; A61B5/055 380

According to International Patent Classification (IPC) or to both national classification and IPC

| B. | FIELDS SEARCHED |
|---|---|

Minimum documentation searched (classification system followed by classification symbols)

A61B 5/055, A61B 6/00 - 6/14, G06T 1/00, G06T 3/00 - 3/60, G06T 5/00 - 5/50, G06T 7/00 - 7/90, G06V10/00 - 20/90, G06V30/418, G06V40/16, G06V40/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 112801992 A (NORTHEASTERN UNIVERSITY) 14 May 2021 (2021-05-14)<br>claim 1 | 1-12 |
| A | CN 111133470 A (DEUTSCHES KREBSFORSCHUNGSZENTRUM STIFTUNG DES OEFFENTLICHEN RECHTS) 08 May 2020 (2020-05-08)<br>paragraphs [0007], [0009]-[0010], [0015], [0019] | 1-12 |
| A | JP 2020-010805 A (DAI NIPPON PRINTING CO., LTD.) 23 January 2020 (2020-01-23)<br>paragraphs [0055], [0058]-[0059], [0062], [0092], fig. 12-16 | 1-12 |

☐ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 December 2023** | **16 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/044155**

| Patent document cited in search report | | | Publication date (day/month/year) | Patent family member(s) | Publication date (day/month/year) |
|---|---|---|---|---|---|
| CN | 112801992 | A | 14 May 2021 | (Family: none) | |
| CN | 111133470 | A | 08 May 2020 | US 2020/0273161 A1 paragraphs [0005], [0007]-[0008], [0013], [0017] WO 2019/057750 A1 EP 3460750 A1 | |
| JP | 2020-010805 | A | 23 January 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018175343 A

- JP 2016007270 A